# FASCICULE DE BREVET EUROPEEN

(11) **EP 1 468 669 B1**
(45) Date de publication et mention de la délivrance du brevet: **28.03.2007**
(21) Numéro de dépôt: 04290587.7
(22) Date de dépôt: 04.03.2004
(51) Int. Cl.: A61K 8/90, A61Q 17/04, A61K 8/36

(54) **Composition photoprotectrice aqueuse comprenant au moins un copolymère diblocs ou triblocs et un 4,4-diarylbutadiène, utilisations**
Wässrige Lichtschutzmittel, welche mindesten ein di- oder triblock Copolymer und 4,4-Diarylbutadien enthalten, und deren Verwendung
An aqueous, sunscreen composition comprising at least a di- or tribloc copolymer and 4,4-diarylbutadien, and use thereof

(30) Priorité: 14.04.2003 FR 0304646
(43) Date de publication de la demande: 20.10.2004
(73) Titulaire: L'ORÉAL, 75008 Paris (FR)
(72) Inventeur: l'Alloret, Florence, 75013 Paris (FR)
(74) Mandataire: Miszputen, Laurent

(56) Documents cités:
- US-A- 4 804 531
- US-A- 6 093 385
- US-A1- 2003 059 391
- US-A1- 2003 059 392

## Description

L'invention concerne une composition photoprotectrice comprenant au moins une phase aqueuse, au moins un polymère hydrosoluble ou hydrodispersible de structure diblocs A-B ou triblocs B-A-B où A est un bloc polymérique hydrosoluble ionique et B est un bloc polymérique hydrophobe et au moins un système filtrant les radiations UV, caractérisée par le fait que le système filtrant comprend au moins un filtre UV-A du type 4,4-diarylbutadiène.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

Les rayons UVA et UVB doivent donc être filtrés et il existe actuellement des compositions cosmétiques protectrices de l'épiderme humain renfermant des filtres UVA et UVB.

Ces compositions antisolaires se présentent soit sous la forme de lotion ou sérum aqueux ne contenant pas de phase grasse soit sous la forme d'une émulsion, de type huile-dans-eau (c'est à dire un support cosmétiquement et/ou dermatologiquement acceptable constitué d'une phase continue dispersante aqueuse et d'une phase discontinue dispersée grasse) ou eau-dans-huile (phase aqueuse dispersée dans une phase grasse continue), qui contient, à des concentrations diverses, un ou plusieurs filtres organiques classiques liposolubles et/ou des filtres organiques classiques hydrosolubles capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV. Dans de telles émulsions, les filtres hydrophiles sont présents dans la phase aqueuse et les filtres lipophiles sont présents dans la phase grasse. Il existe également des émulsions multiples obtenues après dispersion d'une émulsion inverse dans une phase aqueuse.

On connaît dans les demandes de brevet EP1279398 et EP1281395 les polymères hydrosolubles ou hydrodispersibles, de structure diblocs A-B ou triblocs B-A-B, où A est un bloc polymérique hydrosoluble ionique et B un bloc polymérique hydrophobe, sont particulièrement intéressants car ils présentent des propriétés gélifiantes de l'eau à de faibles concentrations (inférieures à 1%) et permettent stabiliser des émulsions sans tensio-actif, ayant de belles textures.

Ces polymères peuvent donc être utilisés dans les principaux supports galéniques suivants :
- les lotions ou sérums aqueux ne comprenant pas de phase grasse, en tant qu'agents gélifiants,
- les émulsions qui sont des dispersions de deux liquides immiscibles tels que l'eau et l'huile, en tant qu'agents émulsionnants et/ou gélifiants. On distingue les émulsions directes comprenant une phase huileuse dispersée dans une phase aqueuse, des émulsions inverses étant des dispersions d'une phase aqueuse dans une phase huileuse. Il existe également des émulsions multiples obtenues après dispersion d'une émulsion inverse dans une phase aqueuse.

Les polymères blocs présentent l'avantage de conduire à des compositions ayant une bonne innocuité vis à vis de la peau car elles sont exemptes de molécules tensio-actives de petites tailles. Ils permettent par ailleurs d'accéder à une plus large gamme de textures que les composés gélifiants couramment utilisés (Polymères hydrophiles réticulés tels que les Carbopols fournis par la société Novéon, Polymères naturels tels que la gomme de xanthane) dont le caractère amphiphile n'est pas suffisant pour introduire des teneurs en huile supérieures à 10%.

Parmi les filtres UV-A organiques disponibles, une famille de composés particulièrement efficaces dans l'UV-A est l'acide benzène 1,4-di(3-méthylidène-10-camphosulfonique) et ses différents sels, décrite notamment dans les demandes de brevets FR-A-2528420 et FR-A-2639347, ils sont capables en effet d'absorber les rayons ultraviolets de longueur d'ondes comprises entre 280 et 400 nm, avec des maxima d'absorption compris entre 320 et 400 nm, en particulier aux alentours de 345 nm.

La demanderesse a constaté que l'introduction de ce type de filtre UVA dans les compositions aqueuses solaires gélifiées et/stabilisées par les polymères hydrosolubles ou hydrodispersibles, de structure diblocs A-B ou triblocs B-A-B peut induire une diminution de leur viscosité ou bien leur déstabilisation.

Il apparaît ainsi nécessaire de disposer de compositions aqueuses à base de polymères hydrosolubles ou hydrodispersibles, de structure diblocs A-B ou triblocs B-A-B qui soient stables dans de large gamme de consistances possibles et qui puissent contenir des filtres organiques actifs dans l'UV-A d'efficacité comparable à celle de l'acide benzène 1,4-di(3-méthylidène-10-camphosulfonique) et ses différents sels sans les inconvénients énumérés ci-dessus.

La Demanderesse a découvert de façon surprenante que des compositions aqueuses solaires comprenant au moins un polymère hydrosoluble ou hydrodispersible, de structure diblocs A-B ou triblocs B-A-B et au moins un filtre UV-A du type 4,4-diarylbutadiène, répondaient à ce besoin.

Dans la suite de la présente description, on entend par « système filtrant les radiations UV » par un agent filtrant les radiations UV constitué soit d'un composé organique ou minéral unique filtrant les radiations UV soit un mélange de plusieurs composés organiques ou minéraux filtrant les radiations UV, par exemple mélange comprenant un filtre UVA et un filtre UVB.

Cette découverte est à la base de la présente invention.

L'un des objet de l'invention est une composition photoprotectrice comprenant au moins une phase aqueuse, au moins un polymère hydrosoluble ou hydrodispersible de structure diblocs A-B ou triblocs B-A-B où A est un bloc polymérique hydrosoluble ionique et B est un bloc polymérique hydrophobe et au moins un système filtrant les radiations UV, caractérisée par le fait que le système filtrant comprend au moins un filtre UV-A du type 4,4-diarylbutadiène.

D'autres caractéristiques, aspects et avantages de l'invention apparaîtront à la lecture de la description détaillée qui va suivre.

Par « stable », on entend que les aspects macroscopique et microscopique de la composition ne sont pas modifiés après 1 mois à la température ambiante.

Par « hydrosoluble ou hydrodispersible », on entend des polymères qui, introduits dans une phase aqueuse à 25°C, à une concentration massique égale à 1%, permettent l'obtention d'une solution macroscopiquement homogène et transparente, c'est à dire ayant une valeur de transmittance maximum de la lumière, à une longueur d'onde égale à 500 nm, à travers un échantillon de 1 cm d'épaisseur, d'au moins 60%, de préférence d'au moins 70%.

Par « polymérique », on entend un bloc dont la masse molaire est supérieure à 400 g/mole, et de préférence supérieure à 800 g/mole.

Par « hydrophobe », on entend un bloc qui, introduit dans un solvant hydrocarboné à 25°C, à une concentration massique égale à 1%, permet l'obtention d'une solution macroscopiquement homogène et transparente, c'est à dire ayant une valeur de transmittance maximum de la lumière, à une longueur d'onde égale à 500 nm, à travers un échantillon de 1 cm d'épaisseur, d'au moins 70%, de préférence d'au moins 80%. Le solvant hydrocarboné a une constante diélectrique mesurée à 25°C, inférieure à 50 ; ce solvant peut être notamment choisi parmi les alcanes tels que le cyclohexane (constante diélectrique : 2,02), les solvants aromatiques tels que l'éthylbenzène (constante diélectrique : 2,4), les cétones telles que la cyclohexanone (constante diélectrique : 18,3), les alcools tels que le cyclohexanol (constante diélectrique : 15,0), les solvants chlorés hydrocarbonés tels que le dichlorométhane (constante diélectrique : 9,08), les amides telles que la diméthylformamide et les esters tels que l'acétate d'éthyl (constante diélectrique : 6,02).

Le bloc hydrosoluble ionique A des polymères de l'invention est obtenu à partir d'un ou plusieurs monomères hydrosolubles (la) ou les sels de ceux-ci, comme par exemple :
- l'acide (méth)acrylique,
- l'acide styrène sulfonique,
- l'acide vinylsulfonique et l'acide (méth)allylsulfonique,
- l'acide vinyl phophonique,
- l'acide maléique,
- l'acide itaconique,
- l'acide crotonique,
- le chlorure de diméthyldiallyl ammonium;
- le chlorure de méthylvinylimidazolium,
- les carboxybétaïnes ou sulfobétaïnes éthyléniques obtenues par exemple par quaternisation de monomères à insaturation éthyléniques comportant une fonction amine, par des sels de sodium d'acide carboxylique à halogène mobile (ex : chloroacétate) ou par des sulfones cycliques (ex : propane sulfone).
- les monomères vinyliques hydrosolubles de formule (A) suivante : 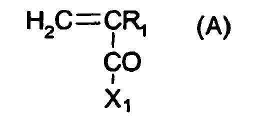
dans laquelle:
- R₁ est choisi parmi H, -CH₃, -C₂H₅ ou -C₃H₇
- X₁ est choisi parmi :
   (i) les oxydes d'alkyle de type -OR₂ où R₂ est un radical hydrocarboné, linéaire ou ramifié, saturé ou insaturé, ayant de 1 à 6 atomes de carbone, substitué par au moins un groupement sulfonique (-SO₃⁻) et/ou sulfate (-SO₄⁻) et/ou phosphate (-PO₄H₂⁻) et/ou un ammonium quaternaire (-N⁺R₃R₄R₅) avec R₃, R₄ et R₅ étant, indépendamment l'un de l'autre, un radical hydrocarboné, linéaire ou ramifié, saturé ou insaturé ayant 1 à 6 atomes de carbone, sous réserve que la somme des atomes de carbone de R₂ + R₃ + R₄ + R₅ ne dépasse pas 6. Le radical R₂ est éventuellement substitué par un atome d'halogène (iode, brome, chlore, fluor); hydroxy (-OH); éther (-O-), amine primaire (-NH₂); amine secondaire (-NHR₆) et/ou amine tertiaire (-NR₆R₇) avec R₆ et R₇ étant, indépendamment l'un de l'autre, un radical hydrocarboné, linéaire ou ramifié, saturé ou insaturé ayant 1 à 6 atomes de carbone, sous réserve que la somme des atomes de carbone de R₂ + R₆ + R₇ ne dépasse pas 6. Citons le méthacrylate de diméthylaminoéthyle quaternisé (MADAME).
   (ii) les groupements -NH₂, -NHR₈ et -NR₈R₉ dans lesquels R₈ et R₉ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₈ + R₉ ne dépasse pas 6, lesdits R₈ et/ou R₉ étant substitués par au moins un groupement sulfonique (-SO₃⁻) et/ou sulfate (-SO₄⁻ ) et/ou phosphate (-PO₄H₂⁻ ) et/ou amine quaternaire (-N⁺R₁₀R₁₁R₁₂) avec R₁₀, R₁₁ et R₁₂ étant, indépendamment l'un de l'autre, un radical hydrocarboné, linéaire ou ramifié, saturé ou insaturé ayant 1 à 6 atomes de carbone, sous réserve que la somme des atomes de carbone de R₈ + R₉ + R₁₀ + R₁₁ + R₁₂ ne dépasse pas 6 . Les radicaux R₈ et/ou R₉ sont éventuellement substitués par un atome d'halogène (iode, brome, chlore, fluor); un groupement hydroxy (-OH); éther (-O-), amine primaire (-NH₂); amine secondaire (-NHR₁₃) et/ou amine tertiaire (-NR₁₃R₁₄) avec R₁₃ et R₁₄ étant, indépendamment l'un de l'autre, un radical hydrocarboné, linéaire ou ramifié, saturé ou insaturé ayant 1 à 6 atomes de carbone, sous réserve que la somme des atomes de carbone de R₈ + R₉+ R₁₃ + R₁₄ ne dépasse pas 6. Citons l'acide acrylamido-2-méthylpropane sulfonique (AMPS), le chlorure de (méth)acrylamido propyl triméthyl ammonium (APTAC et MAPTAC).

Le bloc hydrosoluble ionique A peut également contenir un ou plusieurs monomères hydrosolubles neutres (Ib), comme par exemple :
- le (méth)acrylamide,
- la N-vinylacétamide et la N-méthyl N-vinylacétamide,
- la N-vinylformamide et la N-méthyl N-vinylformamide,
- l'anhydride maléique,
- la vinylamine,
- les N-vinyllactames comportant un groupe alkyl cyclique ayant de 4 à 9 atomes de carbone, tels que la N-vinylpyrrolidone, la N-butyrolactame et la N-vinylcaprolactame,
- l'alcool vinylique de formule CH₂=CHOH,
- les monomères vinyliques hydrosolubles de formule (B) suivante : 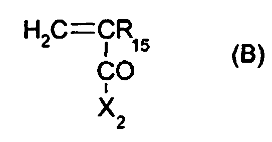
dans laquelle :
- R₁₅ est choisi parmi H, -CH₃, -C₂H₅ ou -C₃H₇
- X₂ est choisi parmi :
   (i) les oxydes d'alkyle de type -OR₁₆ où R₁₆ est un radical hydrocarboné, linéaire ou ramifié, saturé ou insaturé, ayant de 1 à 6 carbones, éventuellement substitué par un atome d'halogène (iode, brome, chlore, fluor); un groupement hydroxy (-OH); éther (-O-) amine primaire (-NH₂); amine secondaire (-NHR₁₇) et/ou amine tertiaire (-NR₁₇R₁₈) avec R₁₇ et R₁₈ étant, indépendamment l'un de l'autre, un radical hydrocarboné, linéaire ou ramifié, saturé ou insaturé ayant 1 à 6 atomes de carbone, sous réserve que la somme des atomes de carbone de R₁₆ + R₁₇ + R₁₈ ne dépasse pas 6 ; Citons par exemple le (méth)acrylate de glycidyle, le méthacrylate d'hydroxyéthyle, et les (méth)acrylates d'éthylène glycol, de diéthylèneglycol ou de polyalkylèneglycol.
   (ii) les groupements -NH₂, -NHR₁₉ et -NR₁₉R₂₀ dans lesquels R₁₉ et R₂₀ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₁₉ + R₂₀ ne dépasse pas 6, lesdits R₁₉ et R₂₀ étant éventuellement substitués par un atome d'halogène (iode, brome, chlore, fluor) ; un groupement hydroxy (-OH); éther (-O-) amine primaire (-NH₂); amine secondaire (-NHR₂₁) et/ou amine tertiaire (-NR₂₁R₂₂) avec R₂₁ et R₂₂ étant, indépendamment l'un de l'autre, un radical hydrocarboné, linéaire ou ramifié, saturé ou insaturé ayant 1 à 6 atomes de carbone, sous réserve que la somme des atomes de carbone de R₁₉ + R₂₀ + R₂₁ + R₂₂ ne dépasse pas 6. Citons le diméthylaminoéthylméthacrylamide.

Le bloc hydrosoluble ionique A des polymères de l'invention peut également contenir un ou plusieurs monomères hydrophobes (Ic), lesdits monomères hydrophobes étant présents en une quantité suffisamment faible pour que le bloc A soit soluble dans l'eau.

Citons par exemple comme monomères hydrophobes (Ic) :
- le styrène et ses dérivés tel que le 4-butylstyrène, l'alpha méthylstyrène et le vinyltoluène,
- l'acétate de vinyl de formule CH₂=CH-OCOCH₃ ;
- les vinyléthers de formule CH₂=CHOR dans laquelle R est un radical hydrocarboné, linéaire ou ramifié, saturé ou insaturé, ayant de 1 à 6 carbones;
- l'acrylonitrile,
- la caprolactone,
- le chlorure de vinyl et le chlorure de vinylidène,
- les dérivés siliconés, conduisant après polymérisation à des polymères siliconés tels que le méthacryloxypropyltris(triméthylsiloxy)silane et les méthacrylamides siliconés,
- les monomères vinyliques hydrophobes de formule (C) suivante : 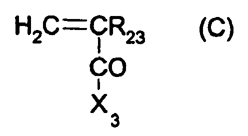
dans laquelle :
- R₂₃ est choisi parmi H, -CH₃, -C₂H₅ ou -C₃H₇
- X₃ est choisi parmi :
   (i) les oxydes d'alkyle de type -OR₂₄ où R₂₄ est un radical hydrocarboné, linéaire ou ramifié, saturé ou insaturé, ayant de 1 à 6 atomes de carbone.
   (ii) les groupements -NH₂, -NHR₂₅ et -NR₂₅R₂₆ dans lesquels R₂₅ et R₂₆ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₂₅+ R₂₆ ne dépasse pas 6. Citons par exemple, le méthacrylate de méthyle, le méthacrylate d'éthyle, le (meth)acrylate de n-butyle, le (meth)acrylate de tertio-butyle, l'acrylate de cyclohexyle et l'acrylate d'isobornyle et l'acrylate d'éthyle 2-hexyle.

Le bloc hydrosoluble ionique A peut également être de la polyéthylèneimine.

Le bloc hydrosoluble ionique A est neutralisé totalement ou partiellement par une base minérale ou organique. Cette base peut être choisie, par exemple, parmi les sels de sodium, ammonium, lithium, calcium, magnésium, ammonium substitué par 1 à 4 groupes alkyle portant de 1 à 15 atomes de carbone, ou encore parmi la mono-, la di-, et la triéthanolamine, l'aminoéthylpropanediol, la N-méthyl-glucamine, et les acides aminés basiques, tels que l'arginine et la lysine, et leurs mélanges.

Le bloc hydrophobe B est obtenu à partir de monomères hydrophobes (Id), comme par exemple :
- le styrène et ses dérivés tel que le 4-butylstyrène, l'alpha méthylstyrène et le vinyltoluène,
- l'acétate de vinyle de formule CH₂=CH-OCOCH₃ ;
- les vinyléthers de formule CH₂=CHOR' dans laquelle R' est un radical hydrocarboné, linéaire ou ramifié, saturé ou insaturé, ayant de 1 à 6 carbones;
- l'acrylonitrile,
- le chlorure de vinyle et le chlorure de vinylidène,
- la caprolactone,
- les alcènes tels que l'éthylène, le propylène, le butylène et le butadiène,
- les dérivés siliconés, conduisant après polymérisation à des polymères siliconés tels que le polydiméthylsiloxane, le méthacryloxypropyltris(triméthylsiloxy)silane et les méthacrylamides siliconés,
- les monomères vinyliques hydrophobes de formule (D) suivante : 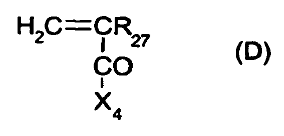
dans laquelle :
- R₂₇ est choisi parmi H, -CH₃, -C₂H₅ ou -C₃H₇
- X₄ est choisi parmi :
   (i) les oxydes d'alkyle de type -OR₂₈ où R₂₈ est un radical hydrocarboné, linéaire ou ramifié, saturé ou insaturé, ayant de 1 à 22 carbones, éventuellement substitué par un atome d'halogène (iode, brome, chlore, fluor); un groupement sulfonique (-SO₃⁻), sulfate (-SO₄⁻ ), phosphate (-PO₄H₂⁻); hydroxy (-OH); éther (-O-) amine primaire (-NH₂); amine secondaire (-NHR₂₉) et/ou amine tertiaire (-NR₂₉R₃₀) ou quaternaire (-N⁺R₂₉R₃₀R₃₁) avec R₂₉, R₃₀ et R₃₁ étant, indépendamment l'un de l'autre, un radical hydrocarboné, linéaire ou ramifié, saturé ou insaturé ayant 1 à 22 atomes de carbone, sous réserve que la somme des atomes de carbone de R₂₈ + R₂₉ + R₃₀ + R₃₁ ne dépasse pas 22. Citons par exemple, le méthacrylate de méthyle, le méthacrylate d'éthyle, le (meth)acrylate de n-butyle, le (meth)acrylate de tertio-butyle, l'acrylate de cyclohexyle et l'acrylate d'isobornyle et l'acrylate d'éthyle 2-hexyle. R₂₈ peut également être un radical perfluoroalkyle, de préférence en C₁₋₁₈; Citons par exemple l'acrylate d'éthyle perfluorooctyle et le (méth)acrylate de trifluorométhyle.
   (ii) les groupements -NH₂, -NHR₃₂ et -NR₃₂R₃₃ dans lesquels R₃₂ et R₃₃ sont indépendamment l'un de l'autre des radicaux hydrocarbonés, linéaires ou ramifiés, saturés ou insaturés ayant 1 à 22 atomes de carbone, sous réserve que le nombre total d'atomes de carbone de R₃₂ + R₃₃ ne dépasse pas 22, lesdits R₃₂ et R₃₃ étant éventuellement substitués par un atome d'halogène (iode, brome, chlore, fluor) ; un groupement hydroxy (-OH); éther (-O-), sulfonique (-SO₃⁻); sulfate (-SO₄⁻ ); phosphate (-PO₄H₂⁻); amine primaire (-NH₂); amine secondaire (-NHR₃₄) et/ou amine tertiaire (-NR₃₄R₃₅) et/ou quaternaire (-N⁺R₃₄R₃₅R₃₆) avec R₃₄, R₃₅ et R₃₆ étant, indépendamment l'un de l'autre, un radical hydrocarboné, linéaire ou ramifié, saturé ou insaturé ayant 1 à 22 atomes de carbone, sous réserve que la somme des atomes de carbone de R₃₂ + R₃₃ + R₃₄ + R₃₅ + R₃₆ ne dépasse pas 22. R₃₂ et R₃₃ peuvent également être des radicaux perfluoroalkyles, de préférence en C₁-₁₈ .

Le bloc hydrophobe B des polymères de l'invention peut également contenir des monomères hydrosolubles (le), les dits monomères hydrosolubles étant présents en une quantité suffisamment faible pour que le bloc B soit hydrophobe.

Citons par exemple comme monomères hydrosolubles (le) et les sels de ceux-ci :
- l'acide (méth)acrylique,
- l'acide styrène sulfonique,
- l'acide vinylsulfonique et l'acide (méth)allylsulfonique,
- l'acide vinyl phophonique,
- l'acide et l'anhydride maléique;
- l'acide itaconique ;
- l'acide crotonique,
- le chlorure de diméthyldiallyl ammonium;
- le chlorure de méthylvinylimidazolium,
- le (méth)acrylamide,
- la N-vinylacétamide et la N-méthyl N-vinylacétamide,
- la N-vinylformamide et la N-méthyl N-vinylformamide,
- les N-vinyllactames comportant un groupe alkyl cyclique ayant de 4 à 9 atomes de carbone, tels que la N-vinylpyrrolidone, la N-butyrolactame et la N-vinylcaprolactame,
- l'alcool vinylique de formule CH₂=CHOH ;
- la 2-vinylpyridine et la 4-vinylpyridine,
- les monomères vinyliques hydrosolubles de formule (E) suivante : 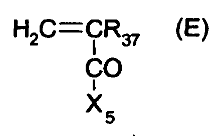
dans laquelle :
- R₃₇ est choisi parmi H, -CH₃, -C₂H₅ ou -C₃H₇
- X₅ est choisi parmi :
   (i) les oxydes d'alkyle de type -OR₃₈ où R₃₈ est un radical hydrocarboné, linéaire ou ramifié, saturé ou insaturé, ayant de 1 à 6 atomes de carbone, éventuellement substitué par un atome d'halogène (iode, brome, chlore, fluor); un groupement sulfonique (-SO₃⁻), sulfate (-SO₄⁻ ), phosphate (-PO₄H₂⁻), hydroxy (-OH); éther (-O-), amine primaire (-NH₂); amine secondaire (-NHR₃₉) et/ou amine tertiaire (-NR₃₉R₄₀) ou un ammonium quaternaire (-N⁺R₃₉R₄₀R₄₁) avec R₃₉, R₄₀ et R₄₁ étant, indépendamment l'un de l'autre, un radical hydrocarboné, linéaire ou ramifié, saturé ou insaturé ayant 1 à 6 atomes de carbone, sous réserve que la somme des atomes de carbone de R₃₈ + R₃₉ + R₄₀ + R₄₁ ne dépasse pas 6. Citons le méthacrylate de diméthylaminoéthyl quaternisé (MADAME), le (méth)acrylate de glycidyle, le méthacrylate d'hydroxyéthyle, et les (méth)acrylates d'éthylène glycol, de diéthylèneglycol ou de polyalkylèneglycol.
   (ii) les groupements -NH₂, -NHR₄₂ et -NR₄₂R₄₃ dans lesquels R₄₂ et R₄₃ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₄₂ + R₄₃ ne dépasse pas 6, lesdits R₄₂ et/ou R₄₃ étant éventuellement substitués par un atome d'halogène (iode, brome, chlore, fluor); un groupement sulfonique (-SO₃⁻); sulfate (-SO₄⁻); phosphate (-PO₄H₂⁻ ); hydroxy (-OH); éther (-O-) amine primaire (-NH₂); amine secondaire (-NHR₄₄) et/ou amine tertiaire (-NR₄₄R₄₅) ou amine quaternaire (-N⁺R₄₄R₄₅R₄₆) avec R₄₄ R₄₅ et R₄₆ étant, indépendamment l'un de l'autre, un radical hydrocarboné, linéaire ou ramifié, saturé ou insaturé ayant 1 à 6 atomes de carbone, sous réserve que la somme des atomes de carbone de R₄₂+ R₄₃ + R₄₄ + R₄₅ + R₄₆ ne dépasse pas 6. Citons la N,N diméthylacrylamide, l'acide acrylamido-2-méthylpropane sulfonique (AMPS), le chlorure de (méth)acrylamido propyl triméthyl ammonium (APTAC et MAPTAC).

Les polymères di-blocs ou triblocs de l'invention ont de préférence une masse molaire comprise entre 1 000 g/mole et 500 000 g/mole, de préférence entre 2 000 g/mole et 100 000 g/mole. Le bloc hydrosoluble ionique A a une masse molaire comprise entre 600 g/mole et 300 000 g/mole, de préférence entre 1 200 g/mole et 60 000 g/mole. Le bloc hydrophobe B a une masse molaire comprise entre 400 g/mole et 200 000 g/mole, de préférence entre 800 g/mole et 40 000 g/mole.

La proportion massique du bloc hydrophile ionique A dans les polymères diblocs A-B de l'invention est de préférence supérieure à 60%, et plus particulièrement supérieure à 70%.

La proportion massique du bloc hydrophile ionique A dans les polymères triblocs B-A-B de l'invention est de préférence supérieure à 50%, et plus particulièrement supérieure à 60%.

En outre, selon un mode préféré de réalisation de l'invention, les polymères diblocs ou triblocs utilisés selon l'invention comme gélifiants ont des blocs polymériques hydrosolubles ioniques A qui sont totalement hydrosolubles, c'est-à-dire complètement exempts de monomère hydrophobe, et des bloc polymériques hydrophobe B totalement hydrophobes, c'est-à-dire complètement exempts de monomère hydrophile.

Les polymères diblocs ou triblocs préférés, comportant des bloc polymériques ioniques A totalement hydrosolubles et des blocs polymériques B totalement hydrophobes présentent l'avantage d'être faciles à synthétiser et de donner une aussi bonne gélification que les autres pour des concentrations moindres.

Parmi les polymères diblocs particulièrement préférés , on peut citer les polymères diblocs Polystyrène/Polyacrylate de sodium et plus particulièrement :
- le polymère diblocs Polystyrène (2000 g/mole)-Polyacrylate de sodium (11500 g/mole) où le bloc ionique hydrosoluble (Polyacrylate de sodium) représente 85,2 % du poids total du polymère diblocs.
- le polymère diblocs Polystyrène (1800 g/mole)-Polyacrylate de sodium (42450 g/mole) où le bloc ionique hydrosoluble (Polyacrylate de sodium) représente 95,9% du poids total du polymère diblocs.
- le polymère diblocs Polystyrène (4300 g/mole) - Polyacrylate de sodium (25460 g/mole) où le bloc ionique hydrosoluble représente 85,55% du poids total du copolymère diblocs.

Parmi les polymères triblocs particulièrement préférés , on peut citer les polymères triblocs Polystyrène/Polyacrylate de sodium/Polystyrène et plus particulièrement :
- le copolymère triblocs Polystyrène (2500 g/mole) - Polyacrylate de sodium (29800 g/mole) - Polystyrène (2500 g/mole) où la quantité de bloc hydrosoluble représente 85,63 % du poids total du copolymère triblocs.

Les polymères de l'invention peuvent être préparés par les procédés de synthèse classiquement utilisés pour obtenir des polymères blocs. Citons les polymérisations vivantes de type anionique ou cationique, et la polymérisation radicalaire contrôlée qui peut être mise en oeuvre suivant différents procédés comme par exemple la voie par transfert d'atomes (Atom Transfert Radical Polymerization ou ATRP), la méthode des radicaux tels que les nitroxydes ou encore la voie par transfert de chaîne réversible avec addition-fragmentation (Radical Addition-Fragmentation chain Transfert) telle que le procédé MADIX (Macromolecular Design via the Interchange of Xanthate). Ces procédés de synthèse peuvent être utilisés pour obtenir les blocs A et B des polymères de l'invention ; ils peuvent également être utilisés pour synthétiser un seul des deux blocs du polymère de l'invention, l'autre bloc étant introduit dans le polymère final par l'intermédiaire de l'amorceur utilisé ou bien par réaction de couplage entre les blocs A et B.

La concentration massique du polymère dans la composition varie de préférence de 0,01% à 30%,en poids de préférence entre 0,1% et 20% en poids par rapport au poids total de la composition.

Les composés 4,4-diarylbutadiènes conformes à l'invention sont choisis de préférence parmi ceux répondant à la formule (II) suivante : 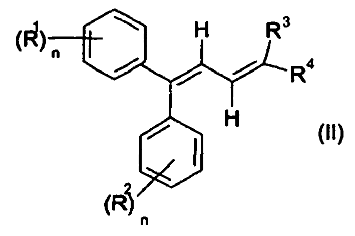 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₂₀,; un radical alcényle en C₂- C₁₀ ; un radical alcoxy en C₁-C₁₂ ; un radical cycloalkyle en C₃-C₁₀ ; un radical cycloalcényle en C₃-C₁₀ ; un radical alcoxycarbonyle en C₁-C₂₀ ; un radical monoalkylamino en C₁-C₁₂ ; un radical dialkylamino en C₁-C₁₂ ; un aryle ; un hétéroaryle ou un substituant hydrosolubilisant choisi parmi un reste carboxylate, sulfonate ou un reste ammonium ;
- R³ désigne un groupe COOR⁵; COR⁵; CONR⁵R⁶ ; CN ; O=S(-R⁵)=O ; O=S(-OR⁵)=O ; R⁷O-P-(-OR⁸)=O; un radical alkyle en C₁-C₂₀ ; un radical alcényle en C₂- C₁₀; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle en C₆-C₁₈ éventuellement substitué ; un hétéroaryle en C₃-C₇ éventuellement substitué;
- R⁴ désigne un groupe COOR⁶; COR⁶; CONR⁵R⁶ ; CN ; O=S(-R⁶)=O ; O=S(-OR⁶)=O ; R⁷O-P-(-OR⁸)=O; un radical alkyle en C₁-C₂₀; un radical alcényle en C₂- C₁₀; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle en C₆-C₁₈ éventuellement substitué ; un hétéroaryle en C₃-C₇ éventuellement substitué;
- les radicaux R⁵ à R⁸, identiques ou différents, désignent hydrogène ; un radical alkyle en C₁-C₂₀ ; un radical alcényle en C₂-C₁₀ ; un radical cycloalkyle en C₃-C₁₀ ; un radical bicycloalkyle en C₇-C₁₀ ; un radical bicycloalcényle en C₃-C₁₀ ; un radical cycloalcényle en C₇-C₁₀ ; un aryle éventuellement substitué ; un hétéroaryle éventuellement substitué ;
- n varie de 1 à 3 ;
les radicaux R³ à R⁸ peuvent former entre eux avec les atomes de carbone auxquels ils sont liés, un noyau en C₅-C₆ pouvant être condensé.

Comme radicaux alkyle en C₁-C₂₀, on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme groupes alcènyle en C₂-C₁₀, on peut citer par exemple : éthènyle, n-propènyle, 1-méthyléthènyle, n-butènyle, 1-méthylpropènyle, 2-méthylpropènyle, 1,1-diméthyléthènyle, n-pentènyle, 1-méthylbutènyle, 2-méthylbutènyle, 3-méthylbutènyle, 2,2-diméthylpropènyle, 1-éthylpropènyle, n-hexènyle, 1,1-diméthylpropènyle, 1,2-diméthylpropènyle, 1-méthylpentènyle, 2-méthylpentènyle, 3-méthylpentènyle, 4-méthylpentènyle, 1,1-diméthylbutènyle, 1,2-diméthylbutènyle, 1,3-diméthylbutènyle, 2,2-diméthylbutènyle, 2,3-diméthylbutènyle, 3,3-diméthylbutènyle, 1-éthylbutènyle, 2-éthylbutènyle, 1,1,2-triméthytpropènyle, 1,2,2-triméthylpropènyle, 1-éthyl-1-méthylpropènyle, 1-éthyl-2-méthylpropènyle, n-heptènyle, n-octènyle, n-nonènyle, n-décènyle.

Comme radicaux alcoxy en C₁-C₁₂, on peut citer : méthoxy, n-propoxy, 1-méthylpropoxy, 1-méthyléthoxy, n-pentoxy, 3-méthylbutoxy, 2,2-diméthylpropoxy, 1-méthyl-1-éthylpropoxy, octoxy, éthoxy, n-propoxy, n-butoxy, 2-méthylpropoxy, 1,1-diméthylpropoxy, hexoxy, heptoxy, 2-éthylhexoxy.

Comme radicaux alcoxycarbonyle en C₁-C₂₀, on peut citer les esters des alcools en C₁-C₂₀.

Comme radicaux monoalkylamino ou dialkylamino en C₁-C₁₂, on peut citer ceux dont le ou les radicaux alkyle sont choisis parmi méthyle, n-propyle, 2-méthylpropyle, 1,1-diméthyléthyle, hexyle, heptyle, 2-éthylhexyle, isopropyle, 1-méthylpropyle, n-pentyle, 3-méthylbutyle, 2,2-diméthylpropyle, 1-méthyl-1-éthylpropyle, octyle.

Comme radicaux cycloalkyles en C₃-C₁₀, on peut citer par exemple : cyclopropyle, cyclobutyle, cyclopentyle, cyclohexyle, cycloheptyle, 1-méthylcyclopropyle, 1-éthylcyclopropyle, 1-propylcyclopropyle, 1-butylcyclopropyle, 1-pentylcyclopropyle, 1-méthyl-1-butylcyclopropyle, 1,2-diméthylcyclypropyle, 1-méthyl-2-éthylcyclopropyle, cyclooctyle, cyclononyle ou cyclodécyle.

Comme radicaux cycloalcènyles en C₃-C₁₀ ayant une ou plusieurs doubles liaisons, on peut citer : cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctétraènyle, cyclononènyle ou cyclodécènyle.

Les radicaux cycloalkyles ou cycloalcényles peuvent comporter un ou plusieurs substituants (de préférence de 1 à 3) choisis par exemple parmi les halogènes comme chlore, fluor ou brome ; cyano ; nitro ; amino ; C₁-C₄-alkylamino ; C₁-C₄ dialkylamino ; C₁-C₄alkyle ; C₁-C₄-alcoxy ; hydroxy ; ils peuvent également comporter de 1 à 3 hétéroatomes comme souffre, oxygène ou azote dont les valences libres peuvent être saturées par un hydrogène ou un radical alkyle en C₁-C₄.

Les groupes bicycloalkyles ou bicycloalcényles sont choisis par exemple parmi les terpènes bicycliques comme les dérivés de pinane, de bornane, de pinène ou de camphre ou d'adamantane.

Les groupes aryles sont de préférence choisis parmi les cycles phényle ou naphtyle, lesquels pouvant comporter un ou plusieurs substituants (de préférence de 1 à 3) chois par exemple parmi halogène comme chlore, fluor ou brome ; cyano ; nitro ; amino ; C₁-C₄-alkylamino ; C₁-C₄dialkylamino ; C₁-C₄alkyle ; C₁-C₄-alcoxy ; hydroxy. On préfère plus particulièrement phényle, méthoxyphényle , naphtyle, thienyle.

Les groupes hétéroaryles comportent en général un ou plusieurs hétéroatomes choisis parmi souffre, oxygène ou azote.

Les groupes hydrosolubilisants sont par exemple des restes carboxy, sulfoxy et plus particulièrement leurs sels avec des cations physiologiquement acceptables comme les sels de métaux alcalins ou les sels de trialkylammonium comme les sels de tri(hydroxyalkyl)ammonium ou de 2-méthylpropan-1-ol-2-ammonium. On peut également citer les groupes ammonium comme les alkylammoniums et leurs formes salifiées avec des anions physiologiquement acceptables.

Les composés de formule (II) sont connus en eux-mêmes et leurs structures et leurs synthèses sont décrites dans les demandes de brevet DE19755649 , EP916335, EP1133980 et EP1133981.

A titre d'exemple de composé de formule (II), on peut citer les composés suivants :

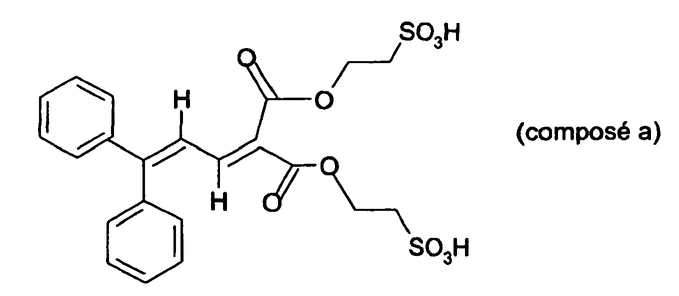

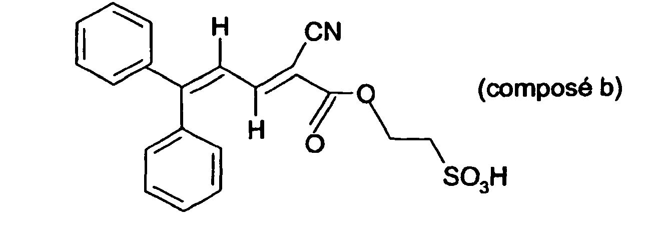

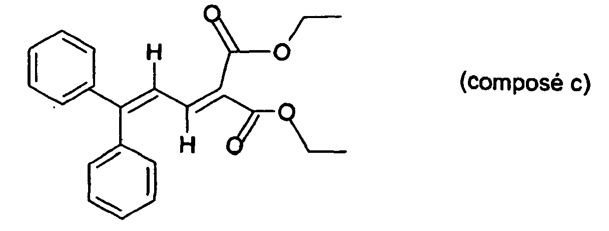

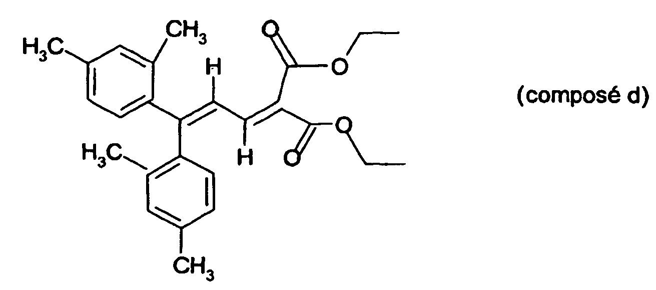

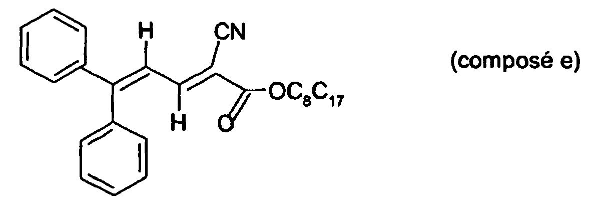

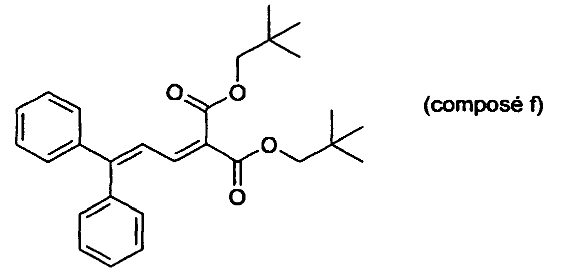

Les composés de formule (II) préférentiels sont ceux pour lesquels
- n=1 ou 2;
- R¹ et R², identiques ou différents, désignent hydrogène, un radical alkyle en C₁-C₂₀,; un radical alcoxy en C₁-C₁₂ ; un radical monoalkylamino en C₁-C₁₂ ; un radical dialkylamino en C₁-C₁₂ ; un substituant hydrosolubilisant choisi parmi un groupe carboxylate, un groupe sulfonate ou un reste ammonium ;
- R³ désigne un groupe COOR⁵; COR⁵ ; CONR⁵R⁶ ; un radical alkyle en C₁-C₂₀ ; un radical cycloalkyle en C₃-C₁₀ ; un radical cycloalcényle en C₃-C₁₀ un radical bicycloalkyle en C₇-C₁₀ ; phényle, naphtyle ou thienyle éventuellement substitué ;
- R⁴ désigne un groupe COOR⁶ ; COR⁶ ; CONR⁵R⁶; un radical alkyle en C₁-C₂₀ ; un radical cycloalkyle en C₃-C₆ ; un radical cycloalcényle en C₃-C₁₀ ; un radical bicycloalkyle en C₇-C₁₀ ; phényle, naphtyle ou thienyle éventuellement substitué ;
- les radicaux R⁵ et R⁶, identiques ou différents, désignent hydrogène ; un radical alkyle en C₁-C₁₂ ; un radical cycloalkyle en C₃-C₁₀ ; un radical cycloalcényle en C₃-C₁₀ ; un radical bicycloalkyle en C₇-C₁₀ ; un radical bicycloalcényle en C₃-C₁₀ ; phényle ou naphtyle éventuellement substitué.

Parmi ces composés, on préfère plus particulièrement ceux pour lesquels
- R¹ et R², identiques ou différents, désignent hydrogène, un radical alkyle en C₁-C₂₀; un radical alcoxy en C₁-C₂₀; un substituant hydrosolubilisant choisi parmi un groupe carboxylate, un groupe sulfonate ou un reste ammonium ;
- R³ désigne un groupe COOR⁵ ; COR⁵; CONR⁵R⁶ ;
- R⁴ désigne un groupe COOR⁶; COR⁶; CONR⁵R⁶ ;
- les radicaux R⁵ et R⁶, identiques ou différents, désignent hydrogène ; un radical alkyle en C₁-C₁₂; un radical cycloalkyle en C₃-C₆ ; un radical cycloalcényle en C₃-C₁₀ ; un radical bicycloalkyle en C₇-C₁₀; un radical bicycloalcényle en C₃-C₁₀ ; phényle ou naphtyle éventuellement substitué.

Selon un mode particulièrement préféré, les composés de formule (II) sont choisis parmi ceux de formule (II') suivante : 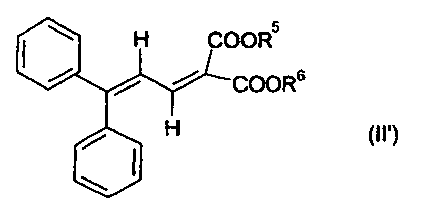 où les radicaux R⁵ et R⁶, identiques ou différents, désignent hydrogène ; un radical alkyle en C₁-C₂₀ ; un radical cycloalkyle en C₃-C₆ ; un radical cycloalcényle en C₃-C₁₀·

Parmi ces composés de formule (II'), on retiendra plus particulièrement le 1,1-dicarboxy-(2'2'-diméthyl-propyl)-4,4-diphénylbutadiène de structure :

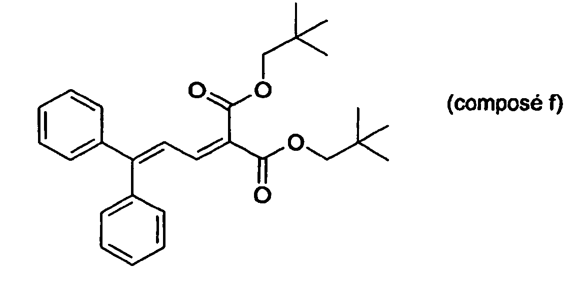

Une autre famille de 4,4-diarylbutadiène pouvant être utilisée dans les émulsions selon l'invention sont ceux répondant à la formule (III) suivante : 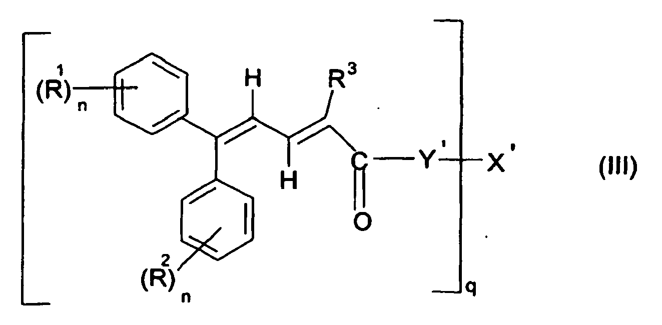 dans laquelle le système diène est de configuration Z,Z ; Z,E ; E,Z ou E,E ou des mélanges desdites configurations et où :
- R¹, R², R³ et n ont les mêmes significations indiquées dans la formule (I) précédente ;
- Y' désigne un groupe -O- ou -NR⁹-
- R⁹ désigne hydrogène ; un radical alkyle en C₁-C₂₀, linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; ; un radical bicycloalcényle en C₇-C₁₀ ; un aryle un hétéroaryle;
- X' désigne un reste de polyol en C₂-C₂₀ linéaire ou ramifié, aliphatique ou cycloaliphatique comprenant de 2 à10 groupes hydroxy et de valence q ; la chaîne carbonée dudit reste pouvant être interrompue par un ou plusieurs atomes de souffre ou d'oxygène ; un ou plusieurs groupes imines ; un ou plusieurs alkylimino en C₁-C₄ ;
- q varie de 2 à 10.

X' est un reste polyol en C₂-C₂₀ contenant de 2 à 10 groupes hydroxyles et notamment :

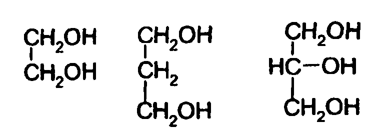

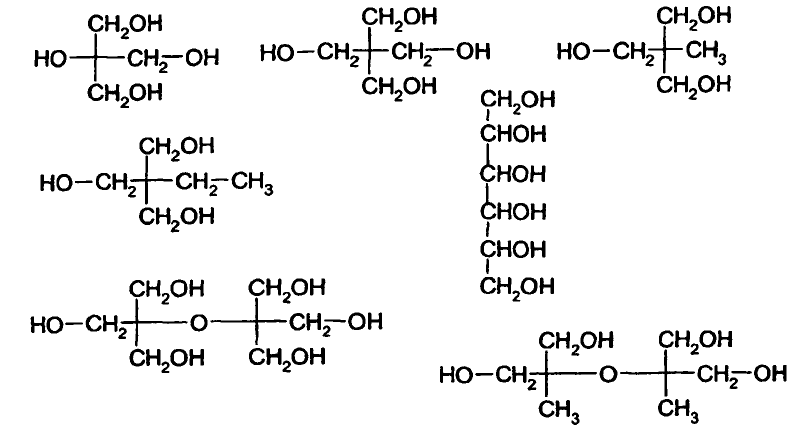

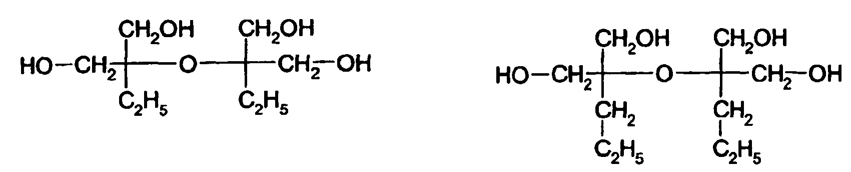

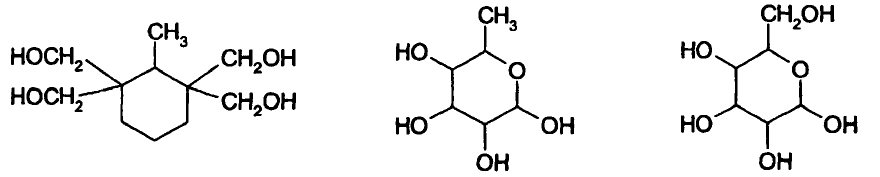

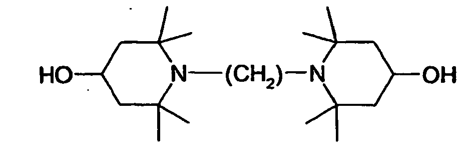

Les composés plus préférentiels de formule (III) sont ceux pour lesquels :
- R¹ et R², identiques ou différents, désignent hydrogène, un radical alkyle en C₁-C₁₂; un radical alcoxy en C₁-C₈; un substituant hydrosolubilisant choisi parmi un groupe carboxylate, un groupe sulfonate ou un reste ammonium ;
- R³ désigne un groupe COOR⁵; CONR⁵R⁶ CN ; un radical cycloalkyle en C₃-C₁₀ ; un radical bicycloalkyle en C₇-C₁₀ ;

- R⁵ et R⁶ , identiques ou différents, désignent un radical alkyle en C₁-C₂₀, linéaire ou ramifié ; un radical cycloalkyle en C₃-C₁₀ ; un radical bicycloalkyle en C₇-C₁₀; naphtyle ou phényle éventuellement substitué ;
- X' désigne un reste de polyol en C₂-C₂₀ comprenant de 2 à 6 groupes hydroxy et plus particulièrement de 2 à 4.

Les composés encore plus préférentiels de formule (II) sont ceux pour lesquels :
- X' désigne un reste d'éthanol ou de pentaerythritol.

Les composés de formule (III) encore plus particulièrement préférés sont choisis parmi

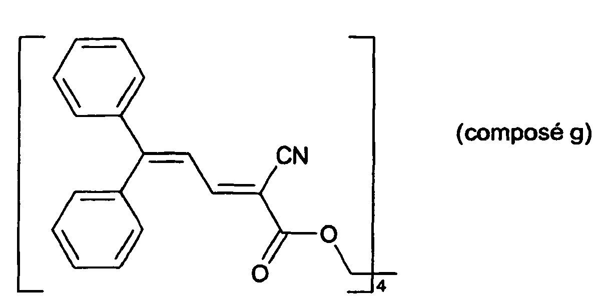

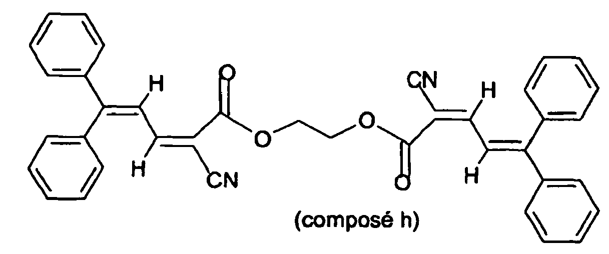

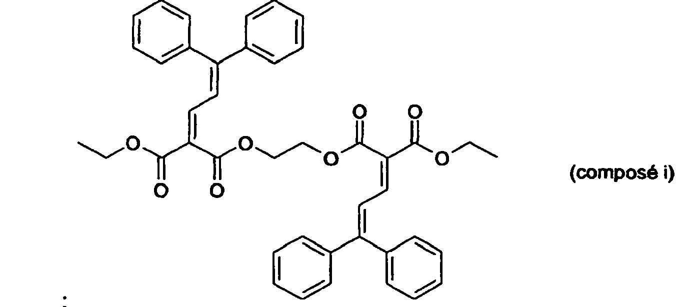

Les composés de formule (III) tels que définis ci-dessus sont connus en eux-mêmes et leurs structures et leurs synthèses sont décrites dans la demande de brevet EP-A-1008586 (faisant partie intégrante du contenu de la description).

Les composés 4,4-diarylbutadiène sont présents de préférence dans la composition dans des proportions allant de 0,1 % à 20% en poids, plus préférentiellement de 1 à 10% en poids par rapport au poids total de la composition.

Les compositions selon l'invention peuvent se présenter sous toutes les formes galéniques comportant une phase aqueuse classiquement utilisées pour une application topique et notamment de lotion ou de sérum sans phase grasse, de dispersions du type émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou des dispersions cire/phase aqueuse. Ces compositions sont préparées selon les méthodes usuelles.

Selon un mode particulier de réalisation de l'invention, les émulsions huile-dans-eau ou eau-dans huile préparées avec les polymères diblocs ou triblocs selon l'invention peuvent comporter seulement 1% en poids ou moins, et même être exemptes de tensioactifs émulsionnants, tout en étant stables au stockage.

La nature de la phase grasse pouvant rentrer dans une composition du type émulsion selon l'invention n'est pas critique et elle peut ainsi être constituée par tous les composés qui sont déjà connus de façon générale comme convenant pour la fabrication d'émulsions de type eau dans huile. En particulier, ces composés peuvent être choisis, seuls ou en mélanges, parmi les différents corps gras, les huiles d'origine végétale, animale ou minérale, les cires naturelles ou synthétiques, et analogues.
Parmi les huiles pouvant rentrer dans la composition de la phase grasse, on peut notamment citer :
- les huiles minérales telles que l'huile de paraffine et l'huile de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle,
- les huiles synthétiques comme par exemple les esters telles que l'huile de purcellin, le myristate de butyle, le myristate d'isopropyle, le myristate de cétyle, le palmitate d'isopropyle, l'adipate d'isopropyle, l'adipate d'éthylhéxyle, le stéarate de butyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle, les isoparaffines et les poly-a-oléfines.

Comme autres huiles utilisables dans les émulsions selon l'invention, on peut encore citer les benzoates d'alcools gras en C12-C15 (Finsolv TN de FINETEX), les éthers, les dérivés lipophiles d'acide aminé tels que le N-lauroylsarcosinate d'isopropyl (Eldew SL-205 d'Ajinomoto), les alcools gras tels que l'alcool laurique, cétylique, myristique, stéarique, palmitique, oléique ainsi que le 2-octyldodécanol, les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés enC10-C18, les huiles fluorées et perfluorées, la lanoline, la lanoline hydrogénée, la lanoline acétylée et enfin les huiles de silicones, volatiles ou non.

Bien entendu, la phase grasse peut également contenir un ou plusieurs adjuvants cosmétiques lipophiles classiques comme par exemple des cires, des gélifiants lipophiles, des tensio-actifs, des particules organiques ou minérales, et notamment ceux qui sont déjà utilisés de manière habituelle dans la fabrication et l'obtention des compositions cosmétiques antisolaires.

De manière classique, la phase aqueuse peut être constituée par de l'eau, ou un mélange d'eau et d'alcool(s) polyhydrique(s) comme par exemple glycérol, propylèneglycol, butylène glycol et sorbitol, ou bien encore un mélange d'eau et d'alcool(s) inférieur(s) hydrosoluble(s) tels que éthanol, isopropanol ou butanol (solution hydroalcoolique).

Les compositions conformes à l'invention peuvent comporter en plus d'autres filtres UV organiques ou inorganiques complémentaires actifs dans l'UVA et/ou l'UVB, hydrosolubles ou liposolubles ou bien insolubles dans les solvants cosmétiques couramment utilisés.

Les filtres organiques complémentaires sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de benzoxazole tels que décrits dans les demandes de brevet EP0832642, EP1027883, EP1300137 et DE10162844 ; les dérivés de méthylène bis-(hydroxyphényl benzotriazole) tels que décrits dans s les demandes US 5,237,071, US 5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 et leurs mélanges.

Comme exemples de filtres organiques actifs dans l'UV-A et/ou l'UV-B, on peut citer désignés ci-dessus sous leur nom INCI :
Dérivés de l'acide para-aminobenzoique:
   PABA,
   Ethyl PABA,
   Ethyl Dihydroxypropyl PABA,
   Ethylhexyl Diméthyl PABA vendu notamment sous le nom « ESCALOL 507 » par ISP, Glyceryl PABA,
   PEG-25 PABA vendu sous le nom « UVINUL P25 » par BASF,
Dérivés salicyliques :
   Homosalate vendu sous le nom « Eusolex HMS » par Rona/EM Industries,
   Ethylhexyl Salicylate vendu sous le nom « NEO HELIOPAN OS » par Haarmann et REIMER,
   Dipropyleneglycol Salicylate vendu sous le nom « DIPSAL » par SCHER,
   TEA Salicylate, vendu sous le nom « NEO HELIOPAN TS » par Haarmann et REIMER,
Dérivés du dibenzoylméthane :
   Butyl Methoxydibenzoylmethane vendu notamment sous le nom commercial « PARSOL 1789 » par HOFFMANN LAROCHE,
   Isopropyl Dibenzoylmethane,
Dérivés cinnamiques :
   Ethylhexyl Methoxycinnamate vendu notamment sous le nom commercial « PARSOL MCX » par HOFFMANN LAROCHE,
   Isopropyl Methoxy cinnamate,
   Isoamyl Methoxy cinnamate vendu sous le nom commercial « NEO HELIOPAN E 1000 » par HAARMANN et REIMER,
   Cinoxate,
   DEA Methoxycinnamate,
   - Diisopropyl Methylcinnamate,
   Glyceryl Ethylhexanoate Dimethoxycinnamate
Dérivés de β,β-diphénylacrylate :
   Octocrylene vendu notamment sous le nom commercial « UVINUL N539 » par BASF,
   Etocrylene, vendu notamment sous le nom commercial « UVINUL N35 » par BASF,
Dérivés de la benzophénone :
   Benzophenone-1 vendu sous le nom commercial « UVINUL 400 » par BASF,
   Benzophenone-2 vendu sous le nom commercial « UVINUL D50 » par BASF
   Benzophenone-3 ou Oxybenzone, vendu sous le nom commercial « UVINUL M40 » par BASF,
   Benzophenone-4 vendu sous le nom commercial « UVINUL MS40 » par BASF, Benzophenone-5
   Benzophenone-6 vendu sous le nom commercial « Helisorb 11 » par Norquay
   Benzophenone-8 vendu sous le nom commercial « Spectra-Sorb UV-24 » par American Cyanamid
   Benzophenone-9 vendu sous le nom commercial« UVINUL DS-49» par BASF, Benzophenone-12,
   le 2-(4-diéthylamino-2-hydroxybenzoyl)-benzoate de n-hexyle
Dérivés du benzylidène camphre:
   3-Benzylidene camphor fabriqué sous le nom « MEXORYL SD» par CHIMEX,
   4-Methylbenzylidene camphor vendu sous le nom « EUSOLEX 6300 » par MERCK ,
   Benzylidene Camphor Sulfonic Acid fabriqué sous le nom « MEXORYL SL» par CHIMEX, Camphor Benzalkonium Methosulfate fabriqué sous le nom « MEXORYL SO » par CHIMEX,
   - Terephthalylidene Dicamphor Sulfonic Acid fabriqué sous le nom « MEXORYL SX » par CHIMEX,
   Polyacrylamidomethyl Benzylidene Camphor fabriqué sous le nom « MESORYL SW » par CHIMEX,
Dérivés de benzimidazole :
   Phenylbenzimidazole Sulfonic Acid vendu notamment sous le nom commercial « EUSOLEX 232 » par MERCK,
   Disodium Phenyl Dibenzimidazole Tetra-sulfonate vendu sous le nom commercial commercial « NEO HELIOPAN AP » par Haarmann et REIMER,
Dérivés de triazine :
   Anisotriazine vendu sous le nom commercial «TINOSORB S » par CIBA SPECIALTY CHEMICALS
   Ethylhexyl triazone vendu notamment sous le nom commercial «UVINUL T150 » par BASF,
   Diethylhexyl Butamido Triazone vendu sous le nom commercial « UVASORB HEB » par SIGMA 3V
   la 2,4,6- tris-(4'amino-benzalmalonate de diisobuty)e)-s-triazine.
Dérivés de benzotriazole :
   Drometrizole Trisiloxane vendu sous le nom « Silatrizole » par RHODIA CHIMIE ,
   Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,
Dérivés anthraniliques :
   Menthyl anthranilate vendu sous le nom commercial commercial « NEO HELIOPAN MA » par Haarmann et REIMER,
Dérivés d'imidazolines :
   Ethylhexyl Dimethoxybenzylidene Dioxoimidazoline Propionate,
Dérivés de benzalmalonate :
   Polyorganosiloxane à fonctions benzalmalonate tel que le polysilicone-15 vendu sous la dénomination commerciale « PARSOL SLX » par HOFFMANN LAROCHE
Dérivés de benzoxazole :
   2,4-bis-[5-1 (diméthylpropyl)benzoxazol-2-yl-(4-phenyl)-imino]-6-(2-ethylhexyl)-imino-1,3,5-triazine vendu sous le nom Uvasorb K2A par Sigma 3V ;
   et leurs mélanges.
Les filtres organiques plus particulièrement préférés sont choisis parmi les composés suivants :
   Ethylhexyl Salicylate,
   Ethylhexyl Methoxycinnamate
   Octocrylene,
   Butyl Methoxydibenzoylmethane
   Phenylbenzimidazole Sulfonic Acid,
   Benzophenone-3,
   Benzophenone-4,
   Benzophenone-5,
   le 2-(4-diéthylamino-2-hydroxybenzoyl)-benzoate de n-hexyle
   4-Methylbenzylidene camphor,
   Terephthalylidene Dicamphor Sulfonic Acid,
   Disodium Phenyl Dibenzimidazole Tetra-sulfonate,
   la 2,4,6-tris-(4'-amino benzalmalonate de diisobutyle)-s-triazine Anisotriazine,
   Ethylhexyl triazone,
   Diethylhexyl Butamido Triazone,
   Methylène bis-Benzotriazolyl Tetramethylbutylphénol
   Drometrizole Trisiloxane
   Polysilicone 15
   2,4-bis-[5-1 (diméthylpropyl)benzoxazol-2-yl-(4-phenyl)-imino]-6-(2-ethylhexyl)-imino-1, 3,5-triazine
   et leurs mélanges.

Les filtres complémentaires inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les filtres complémentaires selon l'invention sont généralement présents dans les compositions selon l'invention à une teneur allant de 0,1 % à 30 % en poids et de préférence de 0,5 à 15 % , en poids, par rapport au poids total de la composition.

Les compositions selon l'invention peuvent également contenir des agents de bronzage et/ou de brunissage artificiels de la peau (agents autobronzants).

Les agents autobronzants sont généralement choisis parmi les composés mono ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342. On utilisera de préférence la DHA.

La DHA peut être utilisée sous forme libre et/ou encapsulée par exemple dans des vésicules lipidiques telle que des liposomes, notamment décrits dans la demande WO 97/25970.

Les agents autobronzants mono ou polycarbonylés sont généralement présents dans les compositions selon l'invention dans des proportions allant de 0,1 à 10% en poids par rapport au poids total de la composition, et de préférence de 0,2 à 8% en poids par rapport au poids total de la composition

Les compositions conformes à la présente invention peuvent comprendre en outre des adjuvants cosmétiques classiques notamment choisis parmi les solvants organiques, les épaississants ioniques ou non ioniques, les adoucissants, les humectants, les opacifiants, les stabilisants, les émollients, les silicones, les agents répulsifs contre les insectes, les parfums, les conservateurs, les tensioactifs, les charges, les actifs, les pigments, les polymères, les propulseurs, les agents alcalinisants ou acidifiants ou tout autre ingrédient habituellement utilisé dans le domaine cosmétique et/ou dermatologique.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Parmi les solvants organiques, on peut citer les alcools et polyols inférieurs.

Parmi les épaississants on peut citer les polymères acryliques réticulés comme les Carbomers fournis par Novéon, les polymères réticulés acrylates/C10-30 alkylacrylates du type Pemulen fournis par Novéon ou le polyacrylate-3 vendu sous le nom Viscophobe DB 1000 par Amerchol) ; les polymères dérivés de l'acide acrylamido 2-méthylpropane sulfonique (Hostacerin AMPS fourni par Clariant, Sépigel 305 fourni par Seppic), les polymères neutres synthétiques tels que la poly N-vinylpyrrolidone, les polysaccharides comme les gommes de guar, de xanthane et les dérivés cellulosiques modifiés ou non comme la gomme de guar hydroxypropylée, la méthylhydroxyéthylcellulose et l'hydroxypropylméthylcellulose.

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Les compositions cosmétiques selon l'invention peuvent par exemple être utilisées comme produit de soin et/ou de protection solaire pour le visage et/ou le corps . de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.
Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Les exemples concrets, mais nullement limitatifs, illustrant l'invention, vont maintenant être donnés.

### Exemples

### Exemple 1 : Laits photoprotecteurs (Emulsions sans tensio-actif stabilisées par un copolymère diblocs A-B)

| **Ingrédients** | **Emulsion 1 (hors invention)** | **Emulsion 2** |
|---|---|---|
| Octyl-méthoxycinnamate | 7 | 7 |
| 1,1-dicarboxy-(2'2'-diméthyl-propyl)-4,4-diphénylbutadiène (composé f) | 0 | 5 |
| Alkyl C₁₂₋₁₅ benzoate | 10 | 10 |
| Glycérine | 3 | 3 |
| Acide terephtalylidène 3,3'-dicamphosulfonique-10,10' | 5 | 0 |
| Polymère diblocs Polystyrène (2000 g/mole) - Polyacrylate de sodium (11500 g/mole). | 3 | 3 |
| Triéthanolamine | 2,3 | 0 |
| Conservateur | 0,3 | 0,3 |
| Eau | qsp 100 | qsp 100 |

Les émulsions 1 et 2 présentent des propriétés photoprotectrices équivalentes.

L'émulsion 1 devient très fluide et présente un phénomène de crèmage après 7 jours à la température ambiante.

L'émulsion 2 selon l'invention reste stable après 7 jours et présente une belle texture de type crème.

### Mode de préparation des émulsions

Le copolymère bloc est solubilisé pendant 2 heures sous agitation dans la phase aqueuse à 60°C ; la solution obtenue est macroscopiquement homogène. Chaque émulsion est préparée par introduction lente de la phase huileuse dans la phase aqueuse sous agitation à l'aide d'un homogénéisateur de type Moritz sous une vitesse d'agitation de 2000 RPM pendant 15 minutes.

### Exemple 2 : Laits photoprotecteurs (Emulsions sans tensio-actif stabilisées par un copolymère triblocs B-A-B)

| **Ingrédients** | **Emulsion 3 (hors invention)** | **Emulsion 4** |
|---|---|---|
| Octyl-méthoxycinnamate | 7 | 7 |
| 1,1-dicarboxy-(2'2'-diméthyl-propyl)-4,4-diphénylbutadiène (composé f) | 0 | 5 |
| Alkyl C₁₂₋₁₅ benzoate | 10 | 10 |
| Glycérine | 3 | 3 |
| Acide terephtalylidène 3,3'-dicamphosulfonique-10,10' | 5 | 0 |
| Polymère triblocs Polystyrène (2500 g/mole) - Polyacrylate de sodium (29800 g/mole) - Polystyrène (2500 g/mole) | 0,6 | 0,6 |
| Triéthanolamine | 2,3 | 0 |
| Conservateur | 0,3 | 0,3 |
| Eau | Qsp 100 | Qsp 100 |

Les émulsions 3 et 4 présentent des propriétés photoprotectrices équivalentes.

L'émulsion 3 devient fluide et se déstabilise macroscopiquement après 24 heures, avec l'apparition d'un phénomène de crèmage.

L'émulsion 4 selon l'invention reste stable après 24 heures et présente une belle texture de type crème.

### Mode de préparation des émulsions

Le copolymère bloc est solubilisé pendant 2 heures sous agitation dans la phase aqueuse à 60°C ; la solution obtenue est macroscopiquement homogène. Chaque émulsion est préparée par introduction lente de la phase huileuse dans la phase aqueuse sous agitation à l'aide d'un homogénéisateur de type Moritz sous une vitesse d'agitation de 2000 RPM pendant 15 minutes.

## Revendications

1. Composition photoprotectrice comprenant au moins une phase aqueuse, au moins un polymère hydrosoluble ou hydrodispersible de structure diblocs A-B ou triblocs B-A-B où A est un bloc polymérique hydrosoluble ionique et B est un bloc polymérique hydrophobe et au moins un système filtrant les radiations UV, **caractérisée par le fait que** le système filtrant comprend au moins un filtre UV-A du type 4,4-diarylbutadiène.

2. Composition selon la revendication 1, où le bloc hydrosoluble ionique A est obtenu à partir d'un ou plusieurs monomères hydrosolubles (la) ou les sels de ceux-ci, choisi parmi
- l'acide (méth)acrytique,
- l'acide styrène sulfonique,
- l'acide vinylsulfonique et l'acide (méth)allylsulfonique,
- l'acide vinyl phophonique,
- l'acide maléique,
- l'acide itaconique,
- l'acide crotonique,
- le chlorure de diméthyldiallyl ammonium;
- le chlorure de méthylvinylimidazolium,
- les carboxybétaïnes ou sulfobétaïnes éthyléniques obtenues par exemple par quatemisation de monomères à insaturation éthyléniques comportant une fonction amine, par des sels de sodium d'acide carboxylique à halogène mobile ou par des sulfones cycliques,
- les monomères vinyliques hydrosolubles de formule (A) suivante : 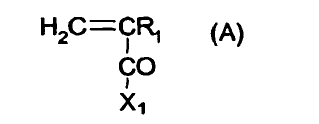
dans laquelle :
- R₁ est choisi parmi H, -CH₃, -C₂H,₅ ou -C₃H₇
- X₁ est choisi parmi :
(i) les oxydes d'alkyle de type -OR₂ où R₂ est un radical hydrocarboné, linéaire ou ramifié, saturé ou insaturé, ayant de 1 à 6 atomes de carbone, substitué par au moins un groupement sulfonique (-SO₃⁻) et/ou sulfate (-SO₄⁻) et/ou phosphate (-PO₄H₂⁻) et/ou un ammonium quaternaire (-N⁺R₃R₄R₅) avec R₃, R₄ et R₅ étant, indépendamment l'un de l'autre, un radical hydrocarboné, linéaire ou ramifié, saturé ou insaturé ayant 1 à 6 atomes de carbone, sous réserve que la somme des atomes de carbone de R₂ + R₃ + R₄ + R₅ ne dépasse pas 6 ; le radical R₂ est éventuellement substitué par un atome d'halogène (iode, brome, chlore, fluor); hydroxy (-OH); éther (-O-), amine primaire (-NH₂); amine secondaire et/ou (-NHR₆), tertiaire (-NR₆R₇) avec R₆ et R₇ étant, indépendamment l'un de l'autre, un radical hydrocarboné, linéaire ou ramifié, saturé ou insaturé ayant 1 à 6 atomes de carbone, sous réserve que la somme des atomes de carbone de R₂ + R₆ + R₇ ne dépasse pas 6 ;
(ii) les groupements -NH₂, -NHR₈ et -NR₈R₉ dans lesquels R₈ et R₉ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₈ + R₉ ne dépasse pas 6, lesdits R₈ et/ou R₉ étant substitués par au moins un groupement sulfonique (-SO₃⁻) et/ou sulfate (-SO₄⁻) et/ou phosphate (-PO₄H₂⁻) et/ou amine quaternaire (-N⁺R₁₀R₁₁R₁₂) avec R₁₀, R₁₁, et R₁₂ étant, indépendamment l'un de l'autre, un radical hydrocarboné, linéaire ou ramifié, saturé ou insaturé ayant 1 à 6 atomes de carbone, sous réserve que la somme des atomes de carbone de R₈ + R₉ + R₁₀ + R₁₁ + R₁₂ ne dépasse pas 6 . Les radicaux R₈ et/ou R₉ sont éventuellement substitués par un atome d'halogène (iode, brome, chlore, fluor); un groupement hydroxy (-OH); éther (-O-), amine primaire (-NH₂); amine secondaire (-NHR₁₃) et/ou amine tertiaire (-NR₁₃R₁₄) avec R₁₃ et R₁₄ étant, indépendamment l'un de l'autre, un radical hydrocarboné, linéaire ou ramifié, saturé ou insaturé ayant 1 à 6 atomes de carbone, sous réserve que la somme des atomes de carbone de R₈ + R₉+ R₁₃ + R₁₄ ne dépasse pas 6.

3. Composition selon la revendication 2, où le bloc hydrosoluble ionique A contient en plus un ou plusieurs monomères hydrosolubles neutres (1b), choisis parmi :
- le (méth)acrylamide,
- la N-vinylacétamide et la N-méthyl N-vinylacétamide,
- la N-vinylformamide et la N-méthyl N-vinylformamide,
- l'anhydride maléique,
- la vinylamine,
- les N-vinyllactames comportant un groupe alkyl cyclique ayant de 4 à 9 atomes de carbone ;
- l'alcool vinylique de formule CH₂=CHOH,
- les monomères vinyliques hydrosolubles de formule (B) suivante : 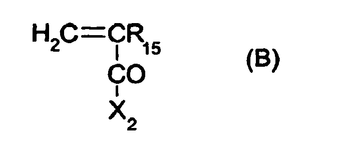
dans laquelle :
- R₁₅ est choisi parmi H, -CH₃, -C₂H₅ ou -C₃H₇
- X₂ est choisi parmi :
(i) les oxydes d'alkyle de type -OR₁₆ où R₁₆ est un radical hydrocarboné, linéaire ou ramifié, saturé ou insaturé, ayant de 1 à 6 carbones, éventuellement substitué par un atome d'halogène (iode, brome, chlore, fluor); un groupement hydroxy (-OH); éther (-O-), amine primaire (-NH₂) amine secondaire et/ou (-NHR₁₇), tertiaire (-NR₁₇R₁₈) avec R₁, et R₁₈ étant, indépendamment l'un de l'autre, un radical hydrocarboné, linéaire ou ramifié, saturé ou insaturé ayant 1 à 6 atomes de carbone, sous réserve que la somme des atomes de carbone de R₁₆ + R₁₇ + R₁₈ ne dépasse pas 6 ;
(ii) les groupements -NH₂, -NHR₁₉ et -NR₁₉R₂₀ dans lesquels R₁₉ et R₂₀ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₁₉ + R₂₀ ne dépasse pas 6, lesdits R₁₉ et R₂₀ étant éventuellement substitués par un atome d'halogène (iode, brome, chlore, fluor) ; un groupement hydroxy (-OH); éther (-O-), amine primaire (-NH₂); amine secondaire (-NHR₂₁) et/ou tertiaire (-NR₂₁R₂₂) avec R₂₁ et R₂₂ étant, indépendamment l'un de l'autre, un radical hydrocarboné, linéaire ou ramifié, saturé ou insaturé ayant 1 à 6 atomes de carbone, sous réserve que la somme des atomes de carbone de R₁₉ + R₂₀ + R₂₁ + R₂₂ ne dépasse pas 6.

4. Composition selon la revendication 2 ou 3, où le bloc hydrosoluble ionique A contient en plus un ou plusieurs monomères hydrophobes (lc), les dits monomères hydrophobes étant présents en une quantité suffisamment faible pour que le bloc A soit soluble dans l'eau.

5. Composition selon la revendication 4, où les monomères hydrophobes (Ic) sont choisis parmi:
- le styrène et ses dérivés ;
- l'acétate de vinyle de formule CH₂=CH-OCOCH₃ ;
- les vinyléthers de formule CH₂=CHOR dans laquelle R est un radical hydrocarboné, linéaire ou ramifié, saturé ou insaturé, ayant de 1 à 6 carbones;
- l'acrylonitrile,
- la caprolactone,
- le chlorure de vinyle et le chlorure de vinylidène,
- les dérivés siliconés,
- les monomères vinyliques hydrophobes de formule (C) suivante : 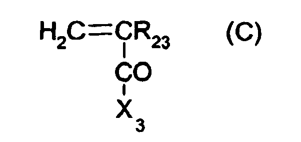
dans laquelle :
- R₂₃ est choisi parmi H, -CH₃, -C₂H₅ ou -C₃H₇
- X₃ est choisi parmi :
(i) les oxydes d'alkyle de type -OR₂₄ où R₂₄ est un radical hydrocarboné, linéaire ou ramifié, saturé ou insaturé, ayant de 1 à 6 atomes de carbone.
(ii) les groupements -NH₂, -NHR₂₅ et -NR₂₅R₂₆ dans lesquels R₂₅ et R₂₆ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₂₅ + R₂₆ ne dépasse pas 6.

6. Composition selon la revendication 1, où le bloc hydrosoluble ionique A peut également être de la polyéthylèneimine.

7. Composition selon l'une quelconque des revendications 1 à 5, où le bloc hydrosoluble ionique A est neutralisé totalement ou partiellement par une base minérale ou organique.

8. Composition selon la revendication 7, où la base minérale ou organique est choisie, parmi les sels de sodium, ammonium, lithium, calcium ou magnésium ; ammonium substitué par 1 à 4 groupes alkyle portant de 1 à 15 atomes de carbone ; la mono-, la di-, et la triéthanolamine, l'aminoéthylpropanediol ; la N-méthyl-glucamine ; les acides aminés basiques et leurs mélanges.

9. Composition selon l'une quelconque des revendications 1 à 8, où le bloc hydrophobe B est obtenu à partir de monomères hydrophobes (Id), choisis parmi :
- le styrène et ses dérivés
- l'acétate de vinyle de formule CH₂=CH-OCOCH₃ ;
- les vinyléthers de formule CH₂=CHOR' dans laquelle R' est un radical hydrocarboné, linéaire ou ramifié, saturé ou insaturé, ayant de 1 à 6 carbones;
- l'acrylonitrile,
- le chlorure de vinyle et le chlorure de vinylidène,
- la caprolactone,
- les alcènes,
- les dérivés siliconés,
- les monomères vinyliques hydrophobes de formule (D) suivante : 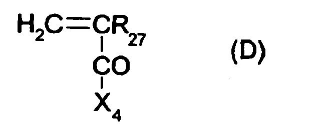
dans laquelle :
- R₂₇ est choisi parmi H, -CH₃, -C₂H₅ ou -C₃H₇
- X₄ est choisi parmi :
(i) les oxydes d'alkyle de type -OR₂₈ où R₂₈ est un radical hydrocarboné, linéaire ou ramifié, saturé ou insaturé, ayant de 1 à 22 carbones, éventuellement substitué par un atome d'halogène (iode, brome, chlore, fluor); un groupement sulfonique (-SO₃⁻), sulfate (-SO₄⁻), phosphate (-PO₄H₂⁻); hydroxy (-OH); éther (-O-), amine primaire (-NH₂); amine secondaire (-NHR₂₉), et/ou amine tertiaire (-NR₂₉R₃₀) ou quaternaire (-N⁺R₂₉R₃₀R₃₁) avec R₂₉, R₃₀ et R₃₁ étant, indépendamment l'un de l'autre, un radical hydrocarboné, linéaire ou ramifié, saturé ou insaturé ayant 1 à 22 atomes de carbone, sous réserve que la somme des atomes de carbone de R₂₈ + R₂₉ + R₃₀ + R₃₁ ne dépasse pas 22 ;
(ii) les groupements -NH₂, -NHR₃₂ et -NR₃₂R₃₃ dans lesquels R₃₂ et R₃₃ sont indépendamment l'un de l'autre des radicaux hydrocarbonés, linéaires ou ramifiés, saturés ou insaturés ayant 1 à 22 atomes de carbone, sous réserve que le nombre total d'atomes de carbone de R₃₂ + R₃₃ ne dépasse pas 22, lesdits R₃₂ et R₃₃ étant éventuellement substitués par un atome d'halogène (iode, brome, chlore, fluor) ; un groupement hydroxy (-OH); éther (-O-), sulfonique (-SO₃⁻); sulfate (-SO₄⁻); phosphate (-PO₄H₂⁻); amine primaire (-NH₂); amine secondaire (-NHR₃₄), et/ou amine tertiaire (-NR₃₄R₃₅) et/ou quaternaire (-N+R₃₄R₃₅R₃₆) avec R₃₄, R₃₅ et R₃₆ étant, indépendamment l'un de l'autre, un radical hydrocarboné, linéaire ou ramifié, saturé ou insaturé ayant 1 à 22 atomes de carbone, sous réserve que la somme des atomes de carbone de R₃₂ + R₃₃ + R₃₄ + R₃₅ + R₃₆ ne dépasse pas 22. R₃₂ et R₃₃ peuvent également être des radicaux perfluoroalkyles, de préférence en C₁₋₁₈.

10. Composition selon la revendication 9, où le bloc hydrophobe B contient en plus un plusieurs monomères hydrosolubles (le) et leurs sels ; les dits monomères hydrosolubles étant présents en une quantité suffisamment faible pour que le bloc B soit hydrophobe.

11. Composition selon la revendication 9, où les monomères hydrosolubles (le) sont choisies parmi :
- l'acide (méth)acrylique,
- l'acide styrène sulfonique,
- l'acide vinylsulfonique et l'acide (méth)allylsulfonique,
- l'acide vinyl phophonique,
- l'acide et l'anhydride maléique;
- l'acide itaconique ;
- l'acide crotonique,
- le chlorure de diméthyldiallyl ammonium;
- le chlorure de méthylvinylimidazolium,
- le (méth)acrylamide,
- la N-vinylacétamide et la N-méthyl N-vinylacétamide,
- la N-vinylformamide et la N-méthyl N-vinylformamide,
- les N-vinyllactames comportant un groupe alkyl cyclique ayant de 4 à 9 atomes de carbone,
- l'alcool vinylique de formule CH₂=CHOH ;
- la 2-vinylpyridine et la 4-vinylpyridine,
- les monomères vinyliques hydrosolubles de formule (E) suivante : 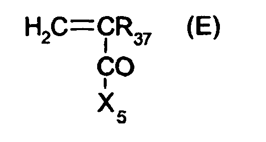
dans laquelle :
- R₃₇ est choisi parmi H, -CH₃, -C₂H₅ ou -C₃H₇
- X₅ est choisi parmi :
- les oxydes d'alkyle de type -OR₃₈ où R₃₈ est un radical hydrocarboné, linéaire ou ramifié, saturé ou insaturé, ayant de 1 à 6 atomes de carbone, éventuellement substitué par un atome d'halogène (iode, brome, chlore, fluor); un groupement sulfonique (-SO₃⁻), sulfate (-SO₄⁻), phosphate (-PO₄H₂⁻), hydroxy (-OH); éther (-O-), amine primaire (-NH₂); amine secondaire (-NHR₃₉) et/ou amine tertiaire (-NR₃₉R₄₀) ou un ammonium quaternaire (-N⁺R₃₉R₄₀R₄₁) avec R₃₉, R₄₀ et R₄₁ étant, indépendamment l'un de l'autre, un radical hydrocarboné, linéaire ou ramifié, saturé ou insaturé ayant 1 à 6 atomes de carbone, sous réserve que la somme des atomes de carbone de R₃₈ + R₃₉ + R₄₀ + R₄₁ ne dépasse pas 6 ;
(ii) les groupements -NH₂, -NHR₄₂ et -NR₄₂R₄₃ dans lesquels R₄₂ et R₄₃ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₄₂ + R₄₃ ne dépasse pas 6, lesdits R₄₂ et/ou R₄₃ étant éventuellement substitués par un atome d'halogène (iode, brome, chlore, fluor); un groupement sulfonique (-SO₃⁻); sulfate (-SO₄⁻); phosphate (-PO₄H₂⁻); hydroxy (-OH); éther (-O-), amine primaire (-NH₂); amine secondaire (-NHR₄₄) et/ou amine tertiaire (-NR₄₄R₄₅) ou amine quaternaire (-N⁺R₄₄R₄₅R₄₆) avec R₄₄ R₄₅ et R₄₆ étant, indépendamment l'un de l'autre, un radical hydrocarboné, linéaire ou ramifié, saturé ou insaturé ayant 1 à 6 atomes de carbone, sous réserve que la somme des atomes de carbone de R₄₂ + R₄₃ + R₄₄ + R₄₅ + R₄₆ ne dépasse pas 6.

12. Composition selon l'une quelconque des revendications 1 à 11, où le polymère diblocs ou triblocs a une masse molaire comprise entre 1 000 g/mole et 500 000 g/mole, de préférence entre 2 000 g/mole et 100 000 g/mole.

13. Composition selon l'une quelconque des revendications 1 à 12, où le bloc hydrosoluble ionique A a une masse molaire comprise entre 600 g/mole et 300 000 g/mole, de préférence entre 1 200 g/mole et 60 000 g/mole.

14. Composition selon l'une quelconque des revendications 1 à 13, où le bloc hydrophobe B a une masse molaire comprise entre 400 g/mole et 200 000 g/mole, de préférence entre 800 g/mole et 40 000 g/mole.

15. Composition selon l'une quelconque des revendications 1 à 14, où la proportion massique du bloc hydrophile ionique A dans les polymères diblocs A-B est supérieure à 60% et plus particulièrement supérieure à 70%.

16. Composition selon l'une quelconque des revendications 1 à 14, où la proportion massique du bloc hydrophile ionique A dans les polymères triblocs B-A-B de l'invention est supérieure à 50%, et plus particulièrement supérieure à 60%.

17. Composition selon l'une quelconque des revendications 1 à 16, où les polymères diblocs ou triblocs ont un bloc polymérique hydrosoluble ionique A totalement hydrosoluble et des blocs polymériques hydrophobe B totalement hydrophobes.

18. Composition selon l'une quelconque des revendications précédentes, où les polymères diblocs sont choisis parmi les polymères diblocs Polystyrène/Polyacrylate de sodium.

19. Composition selon la revendication 18, où le polymère diblocs est choisi parmi :
- le polymère diblocs Polystyrène (2000 g/mole)-Polyacrylate de sodium (11500 g/mole) où le bloc ionique hydrosoluble (Polyacrylate de sodium) représente 85,2 % du poids total du polymère diblocs ;
- le polymère diblocs Polystyrène (1800 g/mole)-Polyacrylate de sodium (42450 g/mole) où le bloc ionique hydrosoluble (Polyacrylate de sodium) représente 95,9% du poids total du polymère diblocs.
- le polymère diblocs Polystyrène (4300 g/mole) - Polyacrylate de sodium (25460 g/mole) où le bloc ionique hydrosoluble représente 85,55% du poids total du copolymère diblocs.

20. Composition selon l'une quelconque des revendications précédentes, où les polymères triblocs sont choisis parmi les polymères triblocs Polystyrène/Polyacrylate de sodium/Polystyrène.

21. Composition selon la revendication 20, où le polymère triblocs est le polymère triblocs Polystyrène (2500 g/mole)-Polyacrylate de sodium (29800 g/mole) - Polystyrène (2500 g/mole où la quantité de bloc hydrosoluble représente 85,63 % du poids total du copolymère triblocs.

22. Composition selon l'une quelconque des revendications précédentes, où la concentration massique du polymère diblocs ou triblocs dans la composition varie de préférence de 0,01% à 30%,en poids de préférence entre 0,1% et 20% en poids par rapport au poids total de la composition.

23. Composition selon l'une quelconque des revendications 1 à 22, où le filtre UV-A du type 4,4-diarylbutadiène répond à la formule (II) suivante : 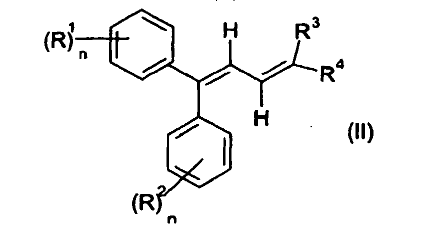 dans laquelle le système diène est de configuration Z,Z; Z,E ; E,Z ou E,E ou des mélanges desdites configurations et où :
- R¹ et R², identiques ou différents, désignent hydrogène, un radical alkyle en C1-C20,; un radical alcényle en C₂ C₁₀ ; un radical alcoxy en C₁-C₁₂ ; un radical cycloalkyle en C₃-C₁₀ ; un radical cycloalcényle en C₃-C₁₀ ; un radical alcoxycarbonyle en C₁-C₂₀ ; un radical monoalkylamino en C₁-C₁₂ ; un radical dialkylamino en C₁-C₁₂ ; un aryle ; un hétéroaryle ou un substituant hydrosolubilisant choisi parmi un reste carboxylate, sulfonate ou un reste ammonium ;
- R³ désigne un groupe COOR⁵ ; COR⁵ ; CONR⁵R⁶; CN ; O=S(-R⁵)=O ; O=S(-OR⁵)=O ; R⁷O-P-(-OR⁸)=O; un radical alkyle en C₁-C₂₀ ; un radical alcényle en C₂- C₁₀ ; un radical cycloalkyle en C₃-C₁₀ ; un radical bicycloalkyle en C₇-C₁₀; un radical cycloalcényle en C₃-C₁₀ ; un radical bicycloalcényle en C₇-C₁₀ ; un aryle en C₆-C₁₈ éventuellement substitué ; un hétéroaryle en C₃-C₇ éventuellement substitué;
- R⁴ désigne un groupe COOR⁶; COR⁶; CONR⁵R⁶ ; CN ; O=S(-R⁶)=O ; O=S(-OR⁶)=O ; R⁷O-P-(-OR⁸)=O; un radical alkyle en C₁-C₂₀ ; un radical alcényle en C₂- C₁₀ ; un radical cycloalkyle en C₃-C₁₀ ; un radical bicycloalkyle en C₇₋C₁₀ ; un radical cycloalcényle en C₃-C₁₀; un radical bicycloalcényle en C₇₋C₁₀; un aryle en C₆-C₁₈ éventuellement substitué ; un hétéroaryle en C₃-C₇ éventuellement substitué;
- les radicaux R⁵ à R⁸, identiques ou différents, désignent hydrogène ; un radical alkyle en C₁-C₂₀ ; un radical alcényle en C₂-C₁₀ ; un radical cycloalkyle en C₃-C₁₀ ; un radical bicycloalkyle en C₇-C₁₀; un radical bicycloalcényle en C₃-C₁₀ ; un radical cycloalcényle en C₇₋C₁₀ ; un aryle éventuellement substitué ; un hétéroaryle éventuellement substitué ;
- n varie de 1 à 3 ;
les radicaux R³ à R⁸ peuvent former entre eux avec les atomes de carbone auxquels ils sont liés, un noyau en C₅-C₆ pouvant être condensé.

24. Composition selon la revendication 23, où le composé de formule (II) est chois parmi ceux pour lesquels
- n=1 ou2;
- R¹ et R², identiques ou différents, désignent hydrogène, un radical alkyle en C₁₋C₂₀; un radical alcoxy en C₁-C₁₂ ; un radical monoalkylamino en C₁-C₁₂ ; un radical dialkylamino en C₁-C₁₂ ; un substituant hydrosolubilisant choisi parmi un groupe carboxylate, un groupe sulfonate ou un reste ammonium ;
- R³ désigne un groupe COOR⁵; COR⁵; CONR⁵R⁶; un radical alkyle en C₁-C₂₀ ; un radical cycloalkyle en C₃-C₁₀ ; un radical cycloalcényle en C₃-C₁₀ un radical bicycloalkyle en C₇-C₁₀ ; phényle, naphtyle ou thienyle éventuellement substitué ;
- R⁴ désigne un groupe COOR⁶; COR⁶; CONR⁵R⁶; un radical alkyle en C₁-C₂₀ ; un radical cycloalkyle en C₃-C₆ ; un radical cycloalcényle en C₃-C₁₀ ; un radical bicycloalkyle en C₇-C₁₀ ; phényle, naphtyle ou thienyle éventuellement substitué ;
- les radicaux R⁵ et R⁶, identiques ou différents, désignent hydrogène; un radical alkyle en C₁-C₁₂ ; un radical cycloalkyle en C₃-C₁₀ ; un radical cycloalcényle en C₃-C₁₀ ; un radical bicycloalkyle en C₇-C₁₀; un radical bicycloalcényle en C₃-C₁₀; phényle ou naphtyle éventuellement substitué.

25. Composition selon la revendication 24, où le composé de formule (II) est chois parmi ceux pour lesquels
- R¹ et R², identiques ou différents, désignent hydrogène, un radical alkyle en C₁-C₂₀; un radical alcoxy en C₁-C₂₀; un substituant hydrosolubilisant choisi parmi un groupe carboxylate, un groupe sulfonate ou un reste ammonium ;
- R³ désigne un groupe COOR⁵; COR⁵, CONR⁵R⁶;
- R⁴ désigne un groupe COOR⁶ ; COR⁶ ; CONR⁵R⁶;
- les radicaux R⁵ et R⁶, identiques ou différents, désignent hydrogène ; un radical alkyle en C₁-C₁₂; un radical cycloalkyle en C₃-C₆ ; un radical cycloalcényle en C₃-C₁₀; un radical bicycloalkyle en C₁-C₁₀; un radical bicycloalcényle en C₃-C₁₀ ; phényle ou naphtyle éventuellement substitué.

26. Composition selon la revendication 25, où le composé de formule (I) est choisi parmi ceux de formule (II') suivante : 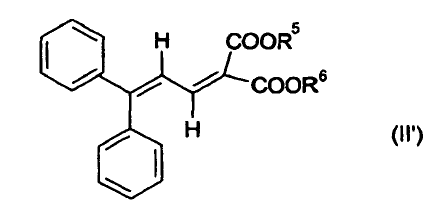 où les radicaux R⁵ et R⁶, identiques ou différents, désignent hydrogène ; un radical alkyle en C₁-C₂₀ ; un radical cycloalkyle en C₃-C₆ ; un radical cycloalcényle en C₃-C₁₀.

27. Composition selon la revendication 26, où le composé de formule (II') est le dérivé 1,1-dicarboxy(2'2'-dimethyl-propyl)-4,4-diphenylbutadiene de structure :

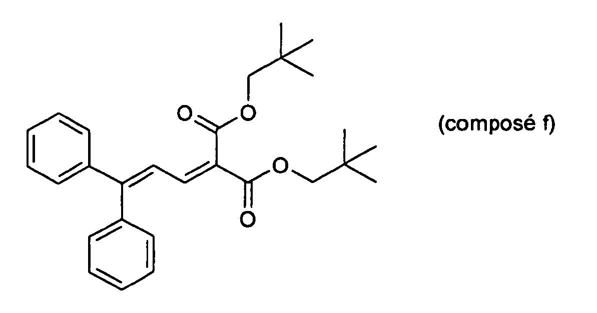

28. Composition selon l'une quelconque des revendications 1 à 22, où le filtre UV-A du type 4,4-diarylbutadiène répond à la formule (III) suivante : 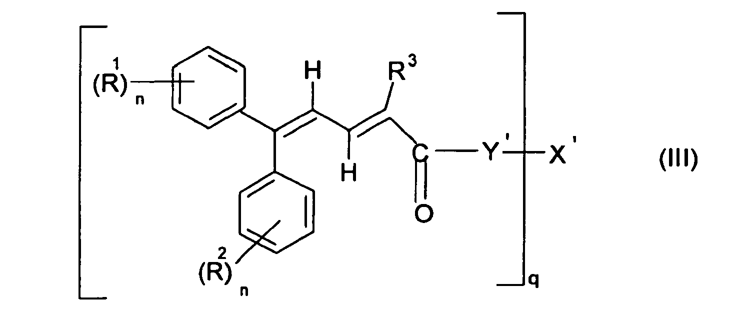 dans laquelle le système diène est de configuration Z,Z ; Z,E ; E,Z ou E,E ou des mélanges desdites configurations et où :
- R¹, R², R³ et n ont les mêmes significations indiquées dans la formule (II) telle que définie dans la revendication 23;
- Y' désigne un groupe -O- ou -NR⁹-
- R⁹ désigne hydrogène ; un radical alkyle en C₁-C₂₀, linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; ; un radical bicycloalcényle en C₇-C₁₀ ; un aryle ; un hétéroaryle;
- X' désigne un reste de polyol en C₂-C₂₀ linéaire ou ramifié, aliphatique ou cycloaliphatique comprenant de 2 à10 groupes hydroxy et de valence q ; la chaîne carbonée dudit reste pouvant être interrompue par un ou plusieurs atomes de souffre ou d'oxygène ; un ou plusieurs groupes imines ; un ou plusieurs alkylimino en C₁-C₄ ;
- q varie de 2 à 10.

29. Composition selon la revendication 28, où le composé de formule (III) est choisi parmi ceux pour lesquels :
- R¹ et R², identiques ou différents, désignent hydrogène, un radical alkyle en C₁-C₁₂ ; un radical alcoxy en C₁-C₈ ; un substituant hydrosolubilisant choisi parmi un groupe carboxylate, un groupe sulfonate ou un reste ammonium ;
- R³ désigne un groupe COOR⁵ CONR⁵R⁶ ; CN ; un radical cycloalkyle en C₃-C₁₀ ; un radical bicycloalkyle en C₇-C₁₀;
- R⁵ et R⁶ , identiques ou différents, désignent un radical alkyle en C₁-C₂₀. linéaire ou ramifié ; un radical cycloalkyle en C₃-C₁₀ ; un radical bicycloalkyle en C₇-C₁₀; naphtyle ou phényle éventuellement substitué ;
- X' désigne un reste de polyol en C₂-C₂₀ comprenant de 2 à 6 groupes hydroxy et plus particulièrement de 2 à 4.

30. Composition selon la revendication 29, où le composé de formule (III) est choisi parmi ceux pour lesquels X' désigne un reste d'éthanol ou de pentaerythritol.

31. Composition selon la revendication 30, où le composé de formule (III) est choisi parmi les composés suivants :

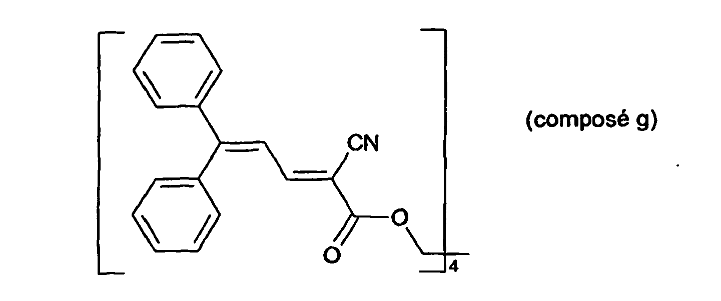

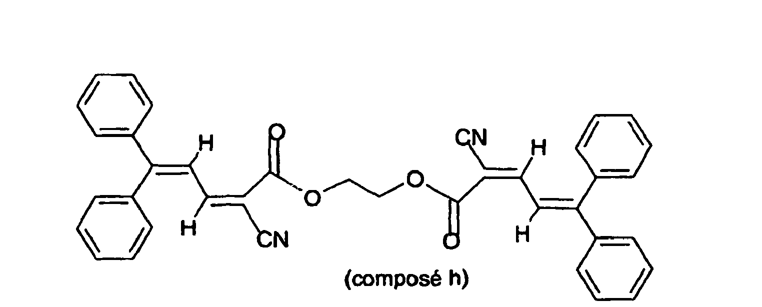

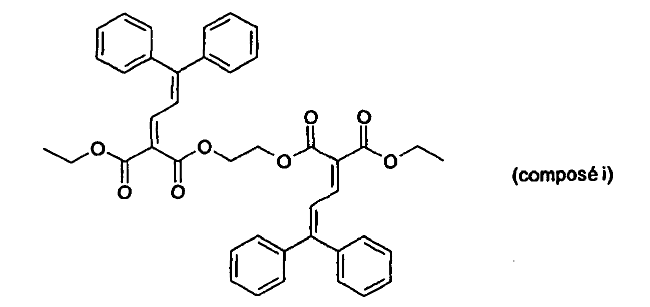

32. Composition selon l'une quelconque des revendications 1 à 31, **caractérisée en ce que** le ou les composés 4,4-diarylbutadiène sont présents dans des proportions allant de 0,1 % à 20% en poids, plus préférentiellement de 1 à 10% en poids par rapport au poids total de l'émulsion.

33. Composition selon l'une quelconque des revendications 1 à 32, **caractérisée en ce qu'**elle contient en outre au moins un filtre solaire organique ou inorganique complémentaire actif dans l'UV-A et/ou l'UV-B, hydrosolubles, liposolubles ou bien insolubles dans les solvants cosmétiques couramment utilisés.

34. Composition selon la revendication 33, ou les filtres organiques complémentaires sont choisis parmi les anthranilates ; les dérivés cinnamiques; les dérivés de dibenzoylméthane ; les dérivés salicyliques, les dérivés du camphre ; les dérivés de triazine ; les dérivés de la benzophénone ; les dérivés de β,β-diphénylacrylate ; les dérivés de benzotriazole ; les dérivés de benzalmalonate ; les dérivés de benzimidazole ; les imidazolines; les dérivés bis-benzoazolyle ; les dérivés de l'acide p-aminobenzoïque (PABA) ; les dérivés de benzoxazole ; les dérivés de méthylène bis-(hydroxyphényl benzotriazole) ; les polymères filtres et silicones filtres ; les dimères dérivés d'α-alkylstyrène et leurs mélanges.

35. Composition selon la revendication 34, ou les filtres organiques complémentaires sont choisis parmi
Ethylhexyl Salicylate,
Ethylhexyl Methoxycinnamate Octocrylene,
Butyl Methoxydibenzoylmethane
Phenylbenzimidazole Sulfonic Acid,
Benzophenone-3,
Benzophenone-4,
Benzophenone-5,
le 2-(4-diéthylamino-2-hydroxybenzoyl)-benzoate de n-hexyle
4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de düsobutyle)-s-triazine Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
2,4-bis-[5-1 (diméthylpropyl)benzoxazol-2-yl-(4-phenyl)-imino]-6-(2-ethylhexyl)-imino-1, 3,5-triazine et leurs mélanges.

36. Composition selon la revendication 33, où les filtres complémentaires inorganiques sont choisis parmi des pigments ou des nanopigments d'oxydes métalliques, enrobés ou non.

37. Composition selon la revendication 36, où les filtres complémentaires inorganiques sont des nanopigments d'oxyde de titane, amorphe ou cristallisé, sous forme rutile et/ou anatase, de fer, de zinc, de zirconium ou de cérium

38. Composition selon l'une quelconque des revendications 1 à 37, **caractérisée en ce qu'**elle contient en outre au moins un agent de bronzage et/ou de brunissage artificiel de la peau.

39. Composition selon l'une quelconque des revendications 1 à 38, **caractérisée en ce qu'**elle contient en outre au moins un adjuvant cosmétique choisi parmi les solvants organiques, les épaississants ioniques ou non ioniques, les adoucissants, les humectants, les actifs, les opacifiants, les stabilisants, les émollients, les silicones, les agents répulsifs contre les insectes, les parfums, les conservateurs, les tensioactifs, les charges, les pigments, les polymères, les propulseurs, les agents alcalinisants ou acidifiants ou tout autre ingrédient habituellement utilisé dans le domaine cosmétique et/ou dermatologique.

40. Composition selon l'une quelconque des revendications 1 à 39, **caractérisée en ce qu'**elle se présente sous forme lotion ou sérum sans phase grasse, d'émulsion huile-dans-eau ou eau-dans-huile ; d'émulsions multiples, de microémulsions, de dispersions vésiculaires de type ionique et/ou non ionique ou des dispersion cire/phase aqueuse.

41. Composition selon l'une quelconque des revendications 1 à 40, **caractérisée en ce qu'**elle se présente sous forme d'émulsion huile-dans-eau ou eau-dans huile comportant au plus 1% en poids par rapport au poids total de la composition en tensioactif émulsionnant.

42. Utilisation d'une composition telle que définie selon l'une quelconque des revendications 1 à 41 pour la fabrication de produits pour le traitement cosmétique de la peau, des lèvres et des cheveux, y compris le cuir chevelu, en particulier pour la protection et/ou le soin de la peau, des lèvres et/ou des cheveux et/ou pour le maquillage de la peau et/ou des lèvres.

## Claims

1. Photoprotective composition comprising at least one aqueous phase, at least one water-soluble or water-dispersible polymer of diblock structure A-B or triblock structure B-A-B in which A is an ionic water-soluble polymeric block and B is a hydrophobic polymeric block and at least one system for screening out UV radiation, **characterized in that** the screening system comprises at least one UV-A-screening agent of the 4,4-diarylbutadiene type.

2. Composition according to Claim 1, in which the ionic water-soluble block A is obtained from one or more water-soluble monomers (Ia) or salts thereof, chosen from:
- (meth)acrylic acid,
- styrenesulfonic acid,
- vinylsulfonic acid and (meth)allylsulfonic acid,
- vinylphosphonic acid,
- maleic acid,
- itaconic acid,
- crotonic acid,
- dimethyldiallylammonium chloride,
- methylvinylimidazolium chloride,
- ethylenic carboxybetaines or sulfobetaines obtained, for example, by quaternization of ethylenically unsaturated monomers containing an amine function, with sodium salts of a carboxylic acid containing a labile halogen or with cyclic sulfones,
- the water-soluble vinyl monomers of formula (A) below: 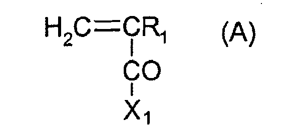
in which:
- R₁ is chosen from H, -CH₃, -C₂H₅ and -C₃H₇,
- X₁ is chosen from:
(i) alkyl oxides of the type -OR₂ in which R₂ is a saturated or unsaturated, linear or branched hydrocarbon-based radical containing from 1 to 6 carbon atoms, substituted with at least one sulfonic (-SO₃⁻) and/or sulfate (-SO₄⁻) and/or phosphate (-PO₄H₂⁻) and/or quaternary ammonium (-N⁺R₃R₄R₅) group with R₃, R₄ and R₅ being, independently of each other, a saturated or unsaturated, linear or branched hydrocarbon-based radical containing 1 to 6 carbon atoms, with the proviso that the sum of the carbon atoms of R₂ + R₃ + R₄ + R₅ does not exceed 6; the radical R₂ is optionally substituted with a halogen atom (iodine, bromine, chlorine or fluorine); hydroxyl (-OH); ether (-O-); primary amine (-NH₂); secondary amine (-NHR₆); and/or tertiary amine (-NR₆R₇) with R₆ and R₇ being, independently of each other, a saturated or unsaturated, linear or branched hydrocarbon-based radical containing 1 to 6 carbon atoms, with the proviso that the sum of the carbon atoms of R₂ + R₆ + R₇ does not exceed 6;
(ii) -NH₂, -NHR₈ and -NR₈R₉ groups in which R₈ and R₉ are, independently of each other, saturated or unsaturated, linear or branched hydrocarbon-based radicals containing 1 to 6 carbon atoms, with the proviso that the total number of carbon atoms of R₈ + R₉ does not exceed 6, the said R₈ and/or R₉ being substituted with at least one sulfonic (-SO₃⁻) and/or sulfate (-SO₄⁻) and/or phosphate (-PO₄H₂⁻) and/or quaternary amine (-N⁺R₁₀R₁₁R₁₀) group with R₁₀, R₁₁ and R₁₂ being, independently of each other, a saturated or unsaturated, linear or branched hydrocarbon-based radical containing 1 to 6 carbon atoms, with the proviso that the sum of the carbon atoms of R₈ + R₉ + R₁₀ + R₁₁ + R₁₂ does not exceed 6. The radicals R₈ and/or R₉ are optionally substituted with a halogen atom (iodine, bromine, chlorine or fluorine); a hydroxyl group (-OH); an ether group (-O-); a primary amine group (-NH₂) ; a secondary amine group (-NHR₁₃) ; and/or a tertiary amine group (-NR₁₃R₁₄) with R₁₃ and R₁₄ being, independently of each other, a saturated or unsaturated, linear or branched hydrocarbon-based radical containing 1 to 6 carbon atoms, with the proviso that the sum of the carbon atoms of R₈ + R₉ + R₁₃ + R₁₄ does not exceed 6.

3. Composition according to Claim 2, in which the ionic water-soluble block A also contains one or more neutral water-soluble monomers (Ib), chosen from:
- (meth) acrylamide,
- N-vinylacetamide and N-methyl-N-vinylacetamide,
- N-vinylformamide and N-methyl-N-vinylformamide,
- maleic anhydride,
- vinylamine,
- N-vinyllactams comprising a cyclic alkyl group containing from 4 to 9 carbon atoms,
- vinyl alcohol of formula CH₂=CHOH,
- the water-soluble vinyl monomers of formula (B) below: 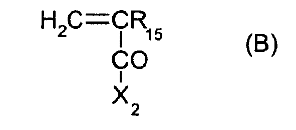
in which:
- R₁₅ is chosen from H, -CH₃, -C₂H₅ and -C₃H₇,
- X₂ is chosen from:
(i) alkyl oxides of the type -OR₁₆ in which R₁₆ is a saturated or unsaturated, linear or branched hydrocarbon-based radical containing from 1 to 6 carbons, optionally substituted with a halogen atom (iodine, bromine, chlorine or fluorine); a hydroxyl group (-OH); an ether group (-O-); a primary amine group (-NH₂); a secondary amine group (-NHR₁₇) ; and/or a tertiary amine group (-NR₁₇R₁₈) with R₁₇ and R₁₈ being, independently of each other, a saturated or unsaturated, linear or branched hydrocarbon-based radical containing 1 to 6 carbon atoms, with the proviso that the sum of the carbon atoms of R₁₆ + R₁₇ + R₁₈ does not exceed 6;
(ii) -NH₂, -NHR₁₉ and -NR₁₉R₂₀ groups in which R₁₉ and R₂₀ are, independently of each other, saturated or unsaturated, linear or branched hydrocarbon-based radicals containing 1 to 6 carbon atoms, with the proviso that the total number of carbon atoms of R₁₉ + R₂₀ does not exceed 6, the said R₁₉ and R₂₀ being optionally substituted with a halogen atom (iodine, bromine, chlorine or fluorine); a hydroxyl group (-OH); an ether group (-O-); a primary amine group (-NH₂); a secondary amine group (-NHR₂₁) ; and/or a tertiary amine group (-NR₂₁R₂₂) with R₂₁ and R₂₂ being, independently of each other, a saturated or unsaturated, linear or branched hydrocarbon-based radical containing 1 to 6 carbon atoms, with the proviso that the sum of the carbon atoms of R₁₉ + R₂₀ + R₂₁ + R₂₂ does not exceed 6.

4. Composition according to Claim 2 or 3, in which the ionic water-soluble block A also contains one or more hydrophobic monomers (Ic), the said hydrophobic monomers being present in an amount low enough for the block A to be water-soluble.

5. Composition according to Claim 4, in which the hydrophobic monomers (Ic) are chosen from:
- styrene and derivatives thereof,
- vinyl acetate of formula CH₂=CH-OCOCH₃,
- vinyl ethers of formula CH₂=CHOR in which R is a saturated or unsaturated, linear or branched hydrocarbon-based radical containing from 1 to 6 carbons,
- acrylonitrile,
- caprolactone,
- vinyl chloride and vinylidene chloride,
- silicone derivatives,
- hydrophobic vinyl monomers of formula (C) below: 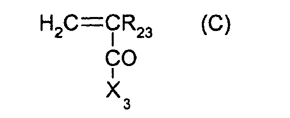
in which:
- R₂₃ is chosen from H, -CH₃, -C₂H₅ and -C₃H₇,
- X₃ is chosen from:
(i) alkyl oxides of the type -OR₂₄ in which R₂₄ is a saturated or unsaturated, linear or branched hydrocarbon-based radical containing from 1 to 6 carbon atoms;
(ii) -NH₂, -NHR₂₅ and -NR₂₅R₂₆ groups in which R₂₅ and R₂₆ are, independently of each other, saturated or unsaturated, linear or branched hydrocarbon-based radicals containing 1 to 6 carbon atoms, with the proviso that the total number of carbon atoms of R₂₅ + R₂₆ does not exceed 6.

6. Composition according to Claim 1, in which the ionic water-soluble block A may also be polyethyleneimine.

7. Composition according to any one of Claims 1 to 5, in which the ionic water-soluble block A is totally or partially neutralized with a mineral or organic base.

8. Composition according to Claim 7, in which the mineral or organic base is chosen from sodium, ammonium, lithium, calcium or magnesium salts; ammonium substituted with 1 to 4 alkyl groups containing from 1 to 15 carbon atoms; monoethanolamine, diethanolamine, triethanolamine, aminoethylpropanediol; N-methylglucamine; basic amino acids, and mixtures thereof.

9. Composition according to any one of Claims 1 to 8, in which the hydrophobic block B is obtained from hydrophobic monomers (Id), chosen from:
- styrene and derivatives thereof,
- vinyl acetate of formula CH₂=CH-OCOCH₃,
- vinyl ethers of formula CH₂=CHOR' in which R' is a saturated or unsaturated, linear or branched hydrocarbon-based radical containing from 1 to 6 carbons,
- acrylonitrile,
- vinyl chloride and vinylidene chloride,
- caprolactone,
- alkenes,
- silicone derivatives,
- hydrophobic vinyl monomers of formula (D) below: 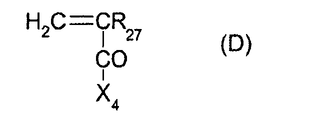
in which:
- R₂₇ is chosen from H, -CH₃, -C₂H₅ and -C₃H₇,
- X₄ is chosen from:
(i) alkyl oxides of the type -OR₂₈ in which R₂₈ is a saturated or unsaturated, linear or branched hydrocarbon-based radical containing from 1 to 22 carbons, optionally substituted with a halogen atom (iodine, bromine, chlorine or fluorine); a sulfonic group (-SO₃⁻) ; a sulfate group (-SO₄⁻) ; a phosphate group (-PO₄H₂⁻) ; a hydroxyl group (-OH); an ether group (-O-); a primary amine group (-NH₂); a secondary amine group (-NHR₂₉) ; and/or a tertiary amine group (-NR₂₉R₃₀) or a quaternary amine group (-N⁺R₂₉R₃₀R₃₁) with R₂₉, R₃₀ and R₃₁ being, independently of each other, a saturated or unsaturated, linear or branched hydrocarbon-based radical containing 1 to 22 carbon atoms, with the proviso that the sum of the carbon atoms of R₂₈ + R₂₉ + R₃₀ + R₃₁ does not exceed 22;
(ii) -NH₂, -NHR₃₂ and -NR₃₂R₃₃ groups in which R₃₂ and R₃₃ are, independently of each other, saturated or unsaturated, linear or branched hydrocarbon-based radicals containing 1 to 22 carbon atoms, with the proviso that the total number of carbon atoms of R₃₂ + R₃₃ does not exceed 22, the said R₃₂ and R₃₃ being optionally substituted with a halogen atom (iodine, bromine, chlorine or fluorine); a hydroxyl group (-OH); an ether group (-O-); a sulfonic group (-SO₃ ⁻); a sulfate group (-SO₄⁻) ; a phosphate group (-PO₄H₂⁻) ; a primary amine group (-NH₂); a secondary amine group (-NHR₃₄) ; and/or a tertiary amine group (-NR₃₄R₃₅) and/or a quaternary amine group (-N⁺R₃₄R₃₅R₃₆) with R₃₄, R₃₅ and R₃₆ being, independently of each other, a saturated or unsaturated, linear or branched hydrocarbon-based radical containing 1 to 22 carbon atoms, with the proviso that the sum of the carbon atoms of R₃₂ + R₃₃ + R₃₄ + R₃₅ + R₃₆ does not exceed 22. R₃₂ and R₃₃ may also be perfluoroalkyl radicals, preferably of C₁₋₁₈.

10. Composition according to Claim 9, in which the hydrophobic block B also contains one or more water-soluble monomers (Ie) and salts thereof; the said water-soluble monomers being present in an amount low enough for the block B to be hydrophobic.

11. Composition according to Claim 9, in which the water-soluble monomers (Ie) are chosen from:
- (meth) acrylic acid,
- styrenesulfonic acid,
- vinylsulfonic acid and (meth)allylsulfonic acid,
- vinylphosphonic acid,
- maleic acid and anhydride,
- itaconic acid,
- crotonic acid,
- dimethyldiallylammonium chloride,
- methylvinylimidazolium chloride,
- (meth)acrylamide,
- N-vinylacetamide and N-methyl-N-vinylacetamide,
- N-vinylformamide and N-methyl-N-vinylformamide,
- N-vinyllactams comprising a cyclic alkyl group containing from 4 to 9 carbon atoms,
- vinyl alcohol of formula CH₂=CHOH,
- 2-vinylpyridine and 4-vinylpyridine,
- water-soluble vinyl monomers of formula (E) below: 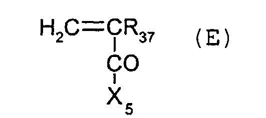
in which:
- R₃₇ is chosen from H, -CH₃, -C₂H₅ and -C₃H₇,
- X₅ is chosen from:
(i) alkyl oxides of the type -OR₃₈ in which R₃₈ is a saturated or unsaturated, linear or branched hydrocarbon-based radical containing from 1 to 6 carbon atoms, optionally substituted with a halogen atom (iodine, bromine, chlorine or fluorine); a sulfonic group (-SO₃⁻) ; a sulfate group (-SO₄⁻) ; a phosphate group (-PO₄H₂⁻) ; a hydroxyl group (-OH); an ether group (-O-) ; a primary amine group (-NH₂); a secondary amine group (-NHR₃₉) ; and/or a tertiary amine group (-NR₃₉R₄₀) or a quaternary ammonium group (-N⁺R₃₉R₄₀R₄₁) with R₃₉, R₄₀ and R₄₁ being, independently of each other, a saturated or unsaturated, linear or branched hydrocarbon-based radical containing 1 to 6 carbon atoms, with the proviso that the sum of the carbon atoms R₃₈ + R₃₉ + R₄₀ + R₄₁ does not exceed 6;
(ii) -NH₂, -NHR₄₂ and -NR₄₂R₄₃ groups in which R₄₂ and R₄₃ are, independently of each other, saturated or unsaturated, linear or branched hydrocarbon-based radicals containing 1 to 6 carbon atoms, with the proviso that the total number of carbon atoms of R₄₂ + R₄₃ does not exceed 6, the said R₄₂ and/or R₄₃ being optionally substituted with a halogen atom (iodine, bromine, chlorine or fluorine); a sulfonic group (-SO₃⁻); a sulfate group (-SO₄⁻); a phosphate group (-PO₄H₂⁻); a hydroxyl group (-OH) ; an ether group (-O-) ; a primary amine group (-NH₂) ; a secondary amine group (-NHR₄₄) ; and/or a tertiary amine group (-NR₄₄R₄₅) or a quaternary amine group (-N⁺R₄₄R₄₅R₄₆) with R₄₄, R₄₅ and R₄₆ being, independently of each other, a saturated or unsaturated, linear or branched hydrocarbon-based radical containing 1 to 6 carbon atoms, with the proviso that the sum of the carbon atoms of R₄₂ + R₄₃ + R₄₄ + R₄₅ + R₄₆ does not exceed 6.

12. Composition according to any one of Claims 1 to 11, in which the diblock or triblock polymer has a molar mass of between 1000 g/mol and 500 000 g/mol and preferably between 2000 g/mol and 100 000 g/mol.

13. Composition according to any one of Claims 1 to 12, in which the ionic water-soluble block A has a molar mass of between 600 g/mol and 300 000 g/mol and preferably between 1200 g/mol and 60 000 g/mol.

14. Composition according to any one of Claims 1 to 13, in which the hydrophobic block B has a molar mass of between 400 g/mol and 200 000 g/mol and preferably between 800 g/mol and 40 000 g/mol.

15. Composition according to any one of Claims 1 to 14, in which the mass proportion of the ionic hydrophilic block A in the diblock polymers A-B is greater than 60% and more particularly greater than 70%.

16. Composition according to any one of Claims 1 to 14, in which the mass proportion of the ionic hydrophilic block A in the triblock polymers B-A-B of the invention is greater than 50%, and more particularly greater than 60%.

17. Composition according to any one of Claims 1 to 16, in which the diblock or triblock polymers have an ionic water-soluble polymeric block A that is totally water-soluble and hydrophobic polymeric blocks B that are totally hydrophobic.

18. Composition according to any one of the preceding claims, in which the diblock polymers are chosen from polystyrene/sodium polyacrylate diblock polymers.

19. Composition according to Claim 18, in which the diblock polymer is chosen from:
- the polystyrene (2000 g/mol)/sodium polyacrylate (11 500 g/mol) diblock polymer in which the water-soluble ionic block (sodium polyacrylate) represents 85.2% of the total weight of the diblock polymer;
- the polystyrene (1800 g/mol)/sodium polyacrylate (42 450 g/mol) diblock polymer in which the water-soluble ionic block (sodium polyacrylate) represents 95.9% of the total weight of the diblock polymer;
- the polystyrene (4300 g/mol)/sodium polyacrylate (25 460 g/mol) diblock polymer in which the water-soluble ionic block represents 85.55% of the total weight of the diblock copolymer.

20. Composition according to any one of the preceding claims, in which the triblock polymers are chosen from polystyrene/sodium polyacrylate/polystyrene triblock polymers.

21. Composition according to Claim 20, in which the triblock polymer is the polystyrene (2500 g/mol)/sodium polyacrylate (29 800 g/mol)/polystyrene (2500 g/mol) triblock polymer in which the quantity of water-soluble block represents 85.63% of the total weight of the triblock copolymer.

22. Composition according to any one of the preceding claims, in which the concentration by mass of the diblock or triblock polymer in the composition preferably ranges from 0.01% to 30% by weight, preferably between 0.1% and 20% by weight relative to the total weight of the composition.

23. Composition according to any one of Claims 1 to 22, in which the UV-A-screening agent of the 4,4-diarylbutadiene type corresponds to formula (II) below: 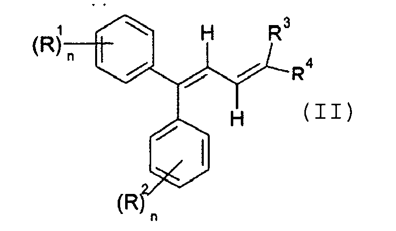 in which the diene system is of the Z,Z; Z,E; E,Z or E,E configuration or mixtures of the said configurations, and in which:
- R¹ and R², which may be identical or different, denote hydrogen, a C₁-C₂₀ alkyl radical; a C₂-C₁₀ alkenyl radical; a C₁-C₁₂ alkoxy radical; a C₃-C₁₀ cycloalkyl radical; a C₃-C₁₀ cycloalkenyl radical; a C₁-C₂₀ alkoxycarbonyl radical; a C₁-C₁₂ monoalkylamino radical; a C₁-C₁₂ dialkylamino radical; an aryl; a heteroaryl or a water-solubilizing substituent chosen from a carboxylate residue, a sulfonate residue and an ammonium residue;
- R³ denotes a group COOR⁵; COR⁵; CONR⁵R⁶; CN; O=S(-R⁵)=O; O=S(-OR⁵ )=O; R⁷O-P-(-OR⁸)=O; a C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an optionally substituted C₆-C₁₈ aryl; an optionally substituted C₃-C₇ heteroaryl;
- R⁴ denotes a group COOR⁶; COR⁶; CONR⁵R⁶; CN; O=S (-R⁶) =O; O=S(-OR⁶)=O; R⁷O-P-(-OR⁸)=O; a C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an optionally substituted C₆-C₁₈ aryl; an optionally substituted C₃-C₇ heteroaryl;
- the radicals R⁵ to R⁸, which may be identical or different, denote hydrogen; a C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ bicycloalkenyl radical; a C₇-C₁₀ cycloalkenyl radical; an optionally substituted aryl; an optionally substituted heteroaryl;
- n ranges from 1 to 3;
the radicals R³ to R⁸ can form, together with the carbon atoms to which they are attached, a C₅-C₆ ring that may be fused.

24. Composition according to Claim 23, in which the compound of formula (II) is chosen from those for which
- n = 1 or 2;
- R¹ and R², which may be identical or different, denote hydrogen, a C₁-C₂₀ alkyl radical; a C₁-C₁₂ alkoxy radical; a C₁-C₁₂ monoalkylamino radical; a C₁-C₁₂ dialkylamino radical; a water-solubilizing substituent chosen from a carboxylate group, a sulfonate group and an ammonium residue;
- R³ denotes a group COOR⁵ ; COR⁵ ; CONR⁵R⁶ ; a C₁-C₂₀ alkyl radical; a C₃-C₁₀ cycloalkyl radical; a C₃-C₁₀ cycloalkenyl radical; a C₇-C₁₀ bicycloalkyl radical; optionally substituted phenyl, naphthyl or thienyl;
- R⁴ denotes a group COOR⁶; COR⁶; CONR⁵R⁶; a C₁-C₂₀ alkyl radical; a C₃-C₆ cycloalkyl radical; a C₃-C₁₀ cycloalkenyl radical; a C₇-C₁₀ bicycloalkyl radical; optionally substituted phenyl, naphthyl or thienyl;
- the radicals R⁵ and R⁶ which may be identical or different, denote hydrogen; a C₁-C₁₂ alkyl radical; a C₃-C₁₀ cycloalkyl radical; a C₃-C₁₀ cycloalkenyl radical; a C₇-C₁₀ bicycloalkyl radical; a C₃-C₁₀ bicycloalkenyl radical; optionally substituted phenyl or naphthyl.

25. Composition according to Claim 24, in which the compound of formula (II) is chosen from those for which
- R¹ and R², which may be identical or different, denote hydrogen, a C₁-C₂₀ alkyl radical; a C₁-C₂₀ alkoxy radical; a water-solubilizing substituent chosen from a carboxylate group, a sulfonate group and an ammonium residue;
- R³ denotes a group COOR⁵; COR⁵; CONR⁵R⁶;
- R⁴ denotes a group COOR⁶; COR⁶; CONR⁵R⁶;
- the radicals R⁵ and R⁶ which may be identical or different, denote hydrogen; a C₁-C₁₂ alkyl radical; a C₃-C₆ cycloalkyl radical; a C₃-C₁₀ cycloalkenyl radical; a C₇-C₁₀ bicycloalkyl radical; a C₃-C₁₀ bicycloalkenyl radical; optionally substituted phenyl or naphthyl.

26. Composition according to Claim 25, in which the compound of formula (II) is chosen from those of below formula (II'): 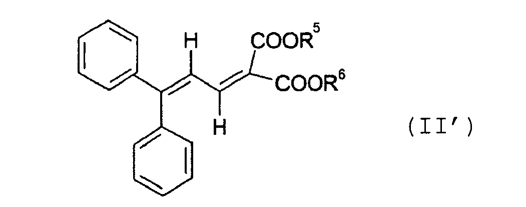 in which the radicals R⁵ and R⁶, which may be identical or different, denote hydrogen; a C₁-C₂₀ alkyl radical; a C₃-C₆ cycloalkyl radical; a C₃-C₁₀ cycloalkenyl radical.

27. Composition according to Claim 26, in which the compound of formula (II') is the 1,1-dicarboxy-(2'2'-dimethylpropyl)-4,4-diphenylbutadiene derivative having the structure:

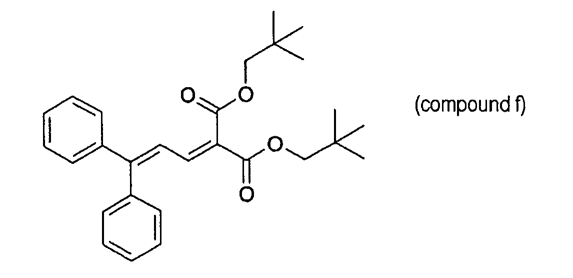

28. Composition according to any one of Claims 1 to 22, in which the UV-A-screening agent of the 4,4-diarylbutadiene type corresponds to formula (III) below: 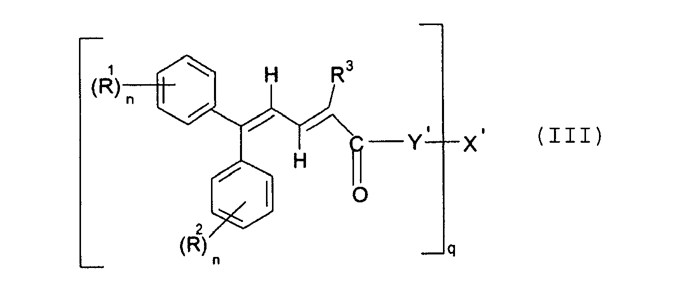 in which the diene system is of the Z,Z; Z,E; E,Z or E,E configuration or mixtures of the said configurations and in which:
- R¹, R² R³ and n have the meanings indicated in formula (II) as defined in Claim 23;
- Y' denotes a group -O- or -NR⁹-;
- R⁹ denotes hydrogen; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; a heteroaryl;
- X' denotes a residue of a linear or branched, aliphatic or cycloaliphatic C₂-C₂₀ polyol comprising from 2 to 10 hydroxyl groups and having the valency q; the carbon chain of the said residue possibly being interrupted with one or more sulfur or oxygen atoms; one or more imine groups; one or more C₁-C₄ alkylimino groups;
- q ranges from 2 to 10.

29. Composition according to Claim 28, in which the compound of formula (III) is chosen from those for which:
- R¹ and R², which may be identical or different, denote hydrogen, a C₁-C₁₂ alkyl radical; a C₁-C₈ alkoxy radical; a water-solubilizing substituent chosen from a carboxylate group, a sulfonate group and an ammonium residue;
- R³ denotes a group COOR⁵; CONR⁵R⁶; CN; a C₃-C₁₀ cycloalkyl radical; a C₇-C₁₀ bicycloalkyl radical;
- R⁵ and R⁶, which may be identical or different, denote a linear or branched C₁-C₂₀ alkyl radical; a C₃-C₁₀ cycloalkyl radical; a C₇-C₁₀ bicycloalkyl radical; optionally substituted naphthyl or phenyl;
- X' denotes a C₂-C₂₀ polyol residue comprising from 2 to 6 and more particularly from 2 to 4 hydroxyl groups.

30. Composition according to Claim 29, in which the compound of formula (III) is chosen from those for which X' denotes an ethanol or pentaerythritol residue.

31. Composition according to Claim 30, in which the compound of formula (III) is chosen from the following compounds:

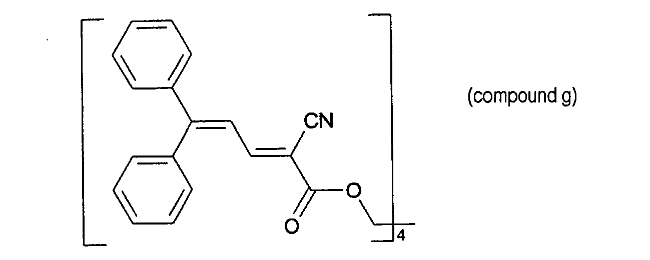

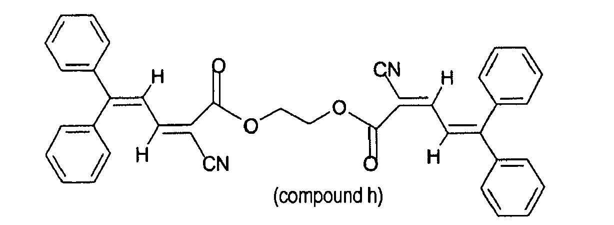

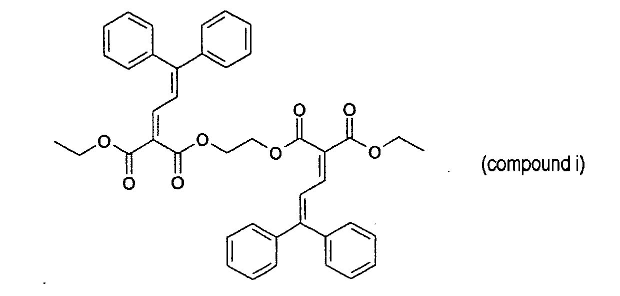

32. Composition according to any one of Claims 1 to 31, **characterized in that** the 4,4-diarylbutadiene compound(s) is (are) present in proportions ranging from 0.1% to 20% by weight and more preferably from 1% to 10% by weight relative to the total weight of the composition.

33. Composition according to any one of Claims 1 to 32, **characterized in that** it also contains at least one additional organic or mineral UV-A-active and/or UV-B-active sunscreen, which are water-soluble, liposoluble or insoluble in the commonly used cosmetic solvents.

34. Composition according to Claim 33, in which the additional organic screening agents are chosen from anthranilates; cinnamic derivatives; dibenzoylmethane derivatives; salicylic derivatives, camphor derivatives; triazine derivatives; benzophenone derivatives; β,β'-diphenylacrylate derivatives; benzotriazole derivatives; benzalmalonate derivatives; benzimidazole derivatives; imidazolines; bis-benzazolyl derivatives; p-aminobenzoic acid (PABA) derivatives; benzoxazole derivatives; methylenebis(hydroxy-phenylbenzotriazole) derivatives; screening polymers and screening silicones; α-alkylstyrene-based dimers, and mixtures thereof.

35. Composition according to Claim 34, in which the additional organic screening agents are chosen from:
Ethylhexyl salicylate,
Ethylhexyl methoxycinnamate,
Octocrylene,
Butylmethoxydibenzoylmethane,
Phenylbenzimidazolesulfonic acid,
Benzophenone-3,
Benzophenone-4,
Benzophenone-5,
n-Hexyl 2-(4-diethylamino-2-hydroxybenzoyl)benzoate,
4-Methylbenzylidenecamphor,
Terephthalylidenedicamphorsulfonic acid,
Disodium phenyldibenzimidazoletetrasulfonate,
2,4,6-Tris(diisobutyl 4'-aminobenzalmalonate)-s-triazine,
Anisotriazine,
Ethylhexyl triazone,
Diethylhexyl butamido triazone,
Methylenebis(benzotriazolyl)tetramethylbutylphenol,
Drometrizole trisiloxane,
Polysilicone-15,
2,4-Bis[5-1-(dimethylpropyl)benzoxazol-2-yl-(4-phenyl)-imino]-6-(2-ethylhexyl)imino-1,3,5-triazine and mixtures thereof.

36. Composition according to Claim 33, in which the additional mineral screening agents are chosen from pigments and nanopigments of metal oxides, which may be coated or uncoated.

37. Composition according to Claim 36, in which the additional mineral screening agents are nanopigments of titanium oxide, which is amorphous or crystallized in rutile and/or anatase form, iron oxide, zinc oxide, zirconium oxide or cerium oxide.

38. Composition according to any one of Claims 1 to 37, **characterized in that** it also contains at least one agent for artificially tanning and/or bronzing the skin.

39. Composition according to any one of Claims 1 to 38, **characterized in that** it also contains at least one cosmetic adjuvant chosen from organic solvents, ionic or nonionic thickeners, softeners, humectants, active agents, opacifiers, stabilizers, emollients, silicones, insect repellents, fragrances, preserving agents, surfactants, fillers, pigments, polymers, propellants, acidifying or basifying agents or any other ingredient usually used in cosmetics and/or dermatology.

40. Composition according to any one of Claims 1 to 39, **characterized in that** it is in the form of a lotion or serum with no fatty phase, an oil-in-water or water-in-oil emulsion; multiple emulsions, microemulsions, vesicular dispersions of the ionic and/or nonionic type, or wax/aqueous phase dispersions.

41. Composition according to any one of Claims 1 to 40, **characterized in that** it is in the form of an oil-in-water or water-in-oil emulsion containing not more than 1% by weight of emulsifying surfactant relative to the total weight of the composition.

42. Use of a composition as defined according to any one of Claims 1 to 41, for manufacturing products for the cosmetic treatment of the skin, the lips and the hair, including the scalp; in particular for protecting and/or caring for the skin, the lips and/or the hair and/or for making up the skin and/or the lips.

## Patentansprüche

1. Zusammensetzung zum Lichtschutz, die mindestens eine wässrige Phase, mindestens ein wasserlösliches oder in Wasser dispergierbares Polymer mit Zweiblockstruktur A-B oder Dreiblockstruktur B-A-B, worin A ein ionischer wasserlöslicher Polymerblock und B ein hydrophober Polymerblock ist, und mindestens ein die UV-Strahlung filterndes System enthält, **dadurch gekennzeichnet, dass** das Filtersystem mindestens ein UV-A-Filter vom 4,4-Diarylbutadientyp umfasst.

2. Zusammensetzung nach Anspruch 1, wobei der ionische wasserlösliche Block A ausgehend von einem oder mehreren wasserlöslichen Monomeren (Ia) oder ihren Salzen gebildet wird, die ausgewählt sind unter
- (Meth)acrylsäure,
- Styrolsulfonsäure,
- Vinylsulfonsäure und (Meth)allylsulfonsäure,
- Vinylphosphonsäure,
- Maleinsäure,
- Itaconsäure,
- Crotonsäure,
- Dimethyldiallylammoniumchlorid,
- Methylvinylimidazoliumchlorid,
- ethylenischen Carboxybetainen oder Sulfobetainen, die beispielsweise durch Quaternisierung von Monomeren mit ethylenisch ungesättigter Bindung, die eine Aminofunktion auf weisen, mit Natriumsalzen einer Carbonsäure mit beweglichem Halogen oder mit cyclischen Sulfonen erhalten werden,
- wasserlöslichen Vinylmonomeren der folgenden Formel (A): 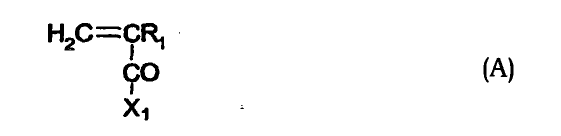
worin in der Formel:
- R₁ unter H, -CH₃, -C₂H₅ oder -C₃H₇ ausgewählt ist,
- X₁ ausgewählt ist unter:
(i) Alkoxygruppen vom Typ -OR₂, wobei R₂ eine geradkettige oder verzweigte, gesättigte oder ungesättigte Kohlenwasserstoffgruppe mit 1 bis 6 Kohlenstoffatomen ist, die mit mindestens einer Sulfonsäuregruppe (-SO₃⁻) und/oder Sulfatgruppe (-SO₄⁻) und/oder Phosphatgruppe (-PO₄H₂⁻) und/oder quartären Ammoniumgruppe (-N⁺R₃R₄R₅) substituiert ist, wobei R₃, R₄ und R₅ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₂ + R₃ + R₄ + R₅ 6 nicht übersteigt; wobei die Gruppe R₂ gegebenenfalls mit einem Halogenatom (Iod, Brom, Chlor, Fluor); einer Hydroxygruppe (-OH); einer Ethergruppe (-O-), einem primären Amin (-NH₂); einem sekundären Amin (-NHR₆) und/oder einem tertiären Amin (-NR₆R₇) substituiert ist, wobei R₆ und R₇ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₂ + R₆ + R₇ 6 nicht übersteigt;
(ii) Gruppen -NH₂, -NHR₈ und -NR₈R₉, worin R₈ und R₉ unabhängig voneinander geradkettige oder verzweigte, gesättigte oder ungesättigte Kohlenwasserstoffgruppen mit 1 bis 6 Kohlenstoffatomen bedeuten, mit der Maßgabe, dass die Gesamtzahl der Kohlenstoffatome R₈ + R₉ 6 nicht übersteigt, wobei R₈ und/oder R₉ mit mindestens einer Sulfonsäuregruppe (-SO₃-) und/oder Sulfatgruppe (-SO₄⁻) und/oder Phosphatgruppe (-PO₄H₂⁻) und/oder quartären Aminogruppe (-N⁺R₁₀R₁₁R₁₂) substituiert sind, wobei R₁₀, R₁₁ und R₁₂ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₈ + R₉ + R₁₀ + R₁₁+ R₁₂ 6 nicht übersteigt. Die Gruppen R₈ und/oder R₉ sind gegebenenfalls mit einem Halogenatom (Iod, Brom, Chlor, Fluor); einer Hydroxygruppe (-OH); einer Ethergruppe (-O-), einer primären Aminogruppe (-NH₂); einer sekundären Aminogruppe (-NHR₁₃) und/oder einer tertiären Aminogruppe (-NR₁₃R₁₄) substituiert, wobei R₁₃ und R₁₄ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von
R₈ + R₉ + R₁₃ + R₁₄ 6 nicht übersteigt.

3. Zusammensetzung nach Anspruch 2, wobei der ionische wasserlösliche Block (A) ferner ein oder mehrere neutrale wasserlösliche Monomere (Ib) enthält, die ausgewählt sind unter:
- (Meth)acrylamid,
- N-Vinylacetamid und N-Methyl-N-vinylacetamid,
- N-Vinylformamid und N-Methyl-N-vinylformamid,
- Maleinsäureanhydrid,
- Vinylamin,
- N-Vinyllactamen, die eine cyclische Alkylgruppe mit 4 bis 9 Kohlenstoffatomen aufweisen,
- dem Vinylalkohol der Formel CH₂=CHOH,
- wasserlöslichen Vinylmonomeren der folgenden Formel (B): 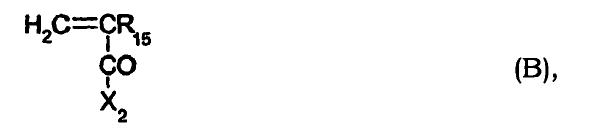
wobei in der Formel:
- R₁₅ unter H, -CH₃, -C₂H₅ oder -C₃H₇ ausgewählt ist,
- X₂ ausgewählt ist unter:
(i) Alkoxygruppen vom Typ -OR₁₆, wobei R₁₆ eine geradkettige oder verzweigte, gesättigte oder ungesättigte Kohlenwasserstoffgruppe mit 1 bis 6 Kohlenstoffatomen ist, die gegebenenfalls mit einem Halogenatom (Iod, Brom, Chlor, Fluor); einer Hydroxygruppe (-OH); einer Ethergruppe (-O-), einer primären Aminogruppe (-NH₂); einer sekundären Aminogruppe (-NHR₁₇) und/oder einer tertiären Aminogruppe (-NR₁₇R₁₈) substituiert ist, wobei R₁₇ und R₁₈ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₁₆ + R₁₇ + R₁₈ 6 nicht übersteigt;
(ii) den Gruppen -NH₂, -NHR₁₉ und -NR₁₉R₂₀, wobei die Gruppen R₁₉ und R₂₀ unabhängig voneinander geradkettige oder verzweigte, gesättigte oder ungesättigte Kohlenwasserstoffgruppen mit 1 bis 6 Kohlenstoffatomen sind, mit der Maßgabe, dass die Gesamtzahl der Kohlenstoffatome von R₁₉ + R₂₀ 6 nicht übersteigt, wobei die Gruppen R₁₉ und R₂₀ gegebenenfalls mit einem Halogenatom (Iod, Brom, Chlor, Fluor); einer Hydroxygruppe (-OH); einer Ethergruppe (-O-), einer primären Aminogruppe (-NH₂); einer sekundären Aminogruppe (-NHR₂₁) und/oder einer tertiären Aminogruppe (-NR₂₁R₂₂) substituiert sind, wobei R₂₁ und R₂₂ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von
R₁₉ + R₂₀ + R₂₁ + R₂₂ 6 nicht übersteigt.

4. Zusammensetzung nach Anspruch 2 oder 3, wobei der ionische wasserlösliche Block A ferner ein oder mehrere hydrophobe Monomere (Ic) enthält, wobei diese hydrophoben Monomere in einer Menge enthalten sind, die ausreichend klein ist, damit der Block A in Wasser löslich ist.

5. Zusammensetzung nach Anspruch 4, wobei die hydrophoben Monomere (Ic) ausgewählt sind unter:
- Styrol und seinen Derivaten;
- dem Vinylacetat der Formel CH₂=CH-OCOCH₃;
- Vinylethern der Formel CH₂=CHOR, wobei R eine geradkettige oder verzweigte, gesättigte oder ungesättigte Kohlenwasserstoffgruppe mit 1 bis 6 Kohlenstoffatomen ist;
- Acrylnitril;
- Caprolacton;
- Vinylchlorid und Vinylidenchlorid;
- Siliconderivaten;
- hydrophoben Vinylmonomeren der folgenden Formel (C): 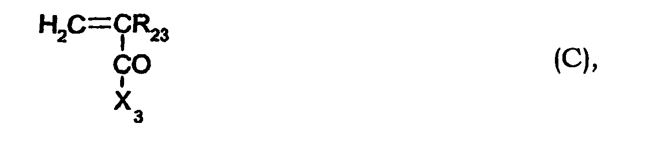
wobei in der Formel:
- R₂₃ unter H, -CH₃, -C₂H₅ oder -C₃H₇ ausgewählt ist,
- X₃ ausgewählt ist unter:
(i) Alkoxygruppen vom Typ -OR₂₄, wobei R₂₄ eine geradkettige oder verzweigte, gesättigte oder ungesättigte Kohlenwasserstoffgruppe mit 1 bis 6 Kohlenstoffatomen ist,
(ii) den Gruppen -NH₂, -NHR₂₅ und -NR₂₅R₂₆, wobei R₂₅ und R₂₆ unabhängig voneinander geradkettige oder verzweigte, gesättigte oder ungesättigte Kohlenwasserstoffgruppen mit 1 bis 6 Kohlenstoffatomen sind, mit der Maßgabe, dass die Gesamtzahl der Kohlenstoffatome von R₂₅ + R₂₆ 6 nicht übersteigt.

6. Zusammensetzung nach Anspruch 1, wobei der ionische wasserlösliche Block A auch das Polyethylenimin sein kann.

7. Zusammensetzung nach einem der Ansprüche 1 bis 5, wobei der ionische wasserlösliche Block A ganz oder teilweise mit einer anorganischen oder organischen Base neutralisiert ist.

8. Zusammensetzung nach Anspruch 7, wobei die anorganische oder organische Base unter den Natriumsalzen, Ammoniumsalzen, Lithiumsalzen, Calciumsalzen oder Magnesiumsalzen; Ammoniumverbindungen, die mit 1 bis 4 Alkylgruppen substituiert sind, die 1 bis 15 Kohlenstoffatome aufweisen; Mono-, Di- und Triethanolamin, Aminoethylpropandiol; N-Methylglucamin; basischen Aminosäuren und deren Gemischen ausgewählt ist.

9. Zusammensetzung nach einem der Ansprüche 1 bis 8, wobei der hydrophobe Block B ausgehend von hydrophoben Monomeren (Id) erhalten wird, die ausgewählt sind unter:
- Styrol und seinen Derivaten;
- dem Vinylacetat der Formel CH₂=CH-OCOCH₃;
- Vinylethern der Formel CH₂=CHOR', wobei R' eine geradkettige oder verzweigte, gesättigte oder ungesättigte Kohlenwasserstoffgruppe mit 1 bis 6 Kohlenstoffatomen ist;
- Acrylnitril,
- Vinylchlorid und Vinylidenchlorid,
- Caprolacton,
- Alkenen,
- Siliconderivaten,
- hydrophoben Vinylmonomeren der folgenden Formel (D): 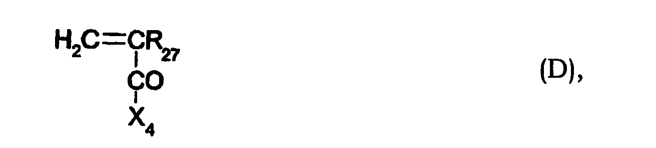
wobei in der Formel:
- R₂₇ unter H, -CH₃, -CH₂H₅ oder -C₃H₇ ausgewählt ist,
- X₄ ausgewählt ist unter:
- Alkoxygruppen vom Typ -OR₂₈, wobei R₂₈ eine geradkettige oder verzweigte, gesättigte oder ungesättigte Kohlenwasserstoffgruppe mit 1 bis 22 Kohlenstoffatomen ist, die gegebenenfalls mit einem Halogenatom (Iod, Brom, Chlor, Fluor); einer Sulfonsäuregruppe (-CO₃⁻), einer Sulfatgruppe (-SO₄⁻), einer Phosphatgruppe (-PO₄H₂⁻); einer Hydroxygruppe (-OH); einer Ethergruppe (-O-), einer primären Aminogruppe (-NH₂); einer sekundären Aminogruppe (-NHR₂₉) und/oder einer tertiären Aminogruppe (NR₂₉R₃₉) oder einer quartären Aminogruppe (-N⁺R₂₉R₃₀R₃₁) substituiert ist, wobei die Gruppen R₂₉, R₃₀ und R₃₁ unabhängig voneinander eine geradkettige oder verzweigte, gesättigte oder ungesättigte Kohlenwasserstoffgruppe mit 1 bis 22 Kohlenstoffatomen bedeuten, mit der Maßgabe, dass die Summe der Kohlenstoffatome von R₂₈ + R₂₉ + R₃₀ + R₃₁ 22 nicht übersteigt;
(ii) den Gruppen -NH₂, -NHR₃₂ und -NR₃₂R₃₃, wobei R₃₂ und R₃₃ unabhängig voneinander geradkettige oder verzweigte, gesättigte oder ungesättigte Kohlenwasserstoffgruppen mit 1 bis 22 Kohlenstoffatomen sind, mit der Maßgabe, dass die Gesamtzahl der Kohlenstoffatome von R₃₂ + R₃₃ 22 nicht übersteigt, wobei die Gruppen R₃₂ + R₃₃ gegebenenfalls mit einem Halogenatom (Iod, Brom, Chlor, Fluor); einer Hydroxygruppe (-OH); einer Ethergruppe (-O-), einer Sulfonsäuregruppe (-SO₃⁻); einer Sulfatgruppe (-SO₄⁻); einer Phosphatgruppe (-PO₄H₂⁻); einer primären Aminogruppe (-NH₂); einer sekundären Aminogruppe (-NHR₃₄) und/oder tertiären Aminogruppe (-NR₃₄R₃₅) und/oder einer quartären Aminogruppe (-N⁺R₃₄R₃₅R₃₆) substituiert sind, wobei R₃₄, R₃₅ und R₃₆ unabhängig voneinander eine geradkettige oder verzweigte, gesättigte oder ungesättigte Kohlenwasserstoffgruppe mit 1 bis 22 Kohlenstoffatomen bedeuten, mit der Maßgabe, dass die Summe der Kohlenstoffatome von R₃₂ + R₃₃ + R₃₄ + R₃₅ + R₃₆ 22 nicht übersteigt, wobei die Gruppen R₃₂ und R₃₃ auch Perfluoralkylgruppen sein können, vorzugsweise mit 1 bis 18 Kohlenstoffatomen.

10. Zusammensetzung nach Anspruch 9, wobei der hydrophobe Block B ferner ein oder mehrere wasserlösliche Monomere (Ie) und deren Salze enthält, wobei die wasserlöslichen Monomere in einer Menge enthalten sind, die ausreichend klein ist, damit der Block B hydrophob ist.

11. Zusammensetzung nach Anspruch 9, wobei die wasserlöslichen Monomere (Ie) ausgewählt sind unter:
- (Meth)acrylsäure,
- Styrolsulfonsäure,
- Vinylsulfonsäure und (Meth)allylsulfonsäure,
- Vinylphosphonsäure,
- Maleinsäureanhydrid,
- Itaconsäure,
- Crotonsäure,
- Dimethyldiallylammoniumchlorid,
- Methylvinylimidazoliumchlorid,
- (Meth)acrylamid,
- N-Vinylacetamid und N-Methyl-N-vinylacetamid,
- N-Vinylformamid und N-Methyl-N-vinylformamid,
- N-Vinyllactamen, die eine cyclische Alkylgruppe mit 4 bis 9 Kohlenstoffatomen aufweisen,
- dem Vinylalkohol der Formel CH₂=CHOH,
- 2-Vinylpyridin und 4-Vinylpyridin,
- wasserlöslichen Vinylmonomeren der folgenden Formel (E): 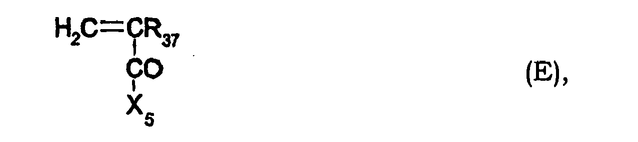
wobei in der Formel:
- R₃₇ unter H, -CH₃, -C₂H₅ oder -C₃H₇ ausgewählt ist,
- X₅ ausgewählt ist unter:
(i)- Alkoxygruppen vom Typ -OR₃₈, wobei R₃₈ eine geradkettige oder verzweigte, gesättigte oder ungesättigte Kohlenwasserstoffgruppe mit 1 bis 6 Kohlenstoffatomen ist, die gegebenenfalls mit einem Halogenatom (Iod, Brom, Chlor, Fluor); einer Sulfonsäuregruppe (-SO₃⁻); einer Sulfatgruppe (-SO₄⁻), einer Phosphatgruppe (-PO₄H₂⁻), einer Hydroxygruppe (-OH); einer Ethergruppe (-O-), einer primären Aminogruppe (-NH₂); einer sekundären Aminogruppe (-NHR₃₉) und/oder einer tertiären Aminogruppe (-NR₃₉R₄₀) oder einer quartären Ammoniumgruppe (-N⁺R₃₉R₄₀R₄₁) substituiert ist, wobei R₃₉ und R₄₀ und R₄₁ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₃₈ + R₃₉ + R₄₀ + R₄₁ 6 nicht übersteigt;
(ii) den Gruppen -NH₂, -NHR₄₂ und -NR₄₂R₄₃, wobei R₄₂ und R₄₃ unabhängig voneinander geradkettige oder verzweigte, gesättigte oder ungesättigte Kohlenwasserstoffgruppen mit 1 bis 6 Kohlenstoffatomen sind, mit der Maßgabe, dass die Gesamtzahl der Kohlenstoffatome von R₄₂ + R₄₃ 6 nicht übersteigt, wobei die Gruppen R₄₂ und/oder R₄₃ gegebenenfalls mit einem Halogenatom (Iod, Brom, Chlor, Fluor); einer Sulfonsäuregruppe (-SO₃⁻); einer Sulfatgruppe (-SO₄⁻); einer Phosphatgruppe (-PO₄H₂⁻); einer Hydroxygruppe (-OH); einer Ethergruppe (-O-), einer primären Aminogruppe (-NH₂); einer sekundären Aminogruppe (-NHR₄₄) und/oder einer tertiären Aminogruppe (-NR₄₄R₄₅) und/oder einer quartären Aminogruppe (-N⁺R₄R₄R₄₆) substituiert sind,
wobei R₄₄, R₄₅ und R₄₆ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₄₂ + R₄₃ + R₄₄ + R₄₅ + R₄₆ 6 nicht übersteigt.

12. Zusammensetzung nach einem der Ansprüche 1 bis 11, wobei das Zweiblockpolymer oder Dreiblockpolymer eine Molmasse von 1 000 bis 500 000 g/mol und vorzugsweise 2 000 bis 100 000 g/mol aufweist.

13. Zusammensetzung nach einem der Ansprüche 1 bis 12, wobei der ionische wasserlösliche Block A eine Molmasse von 600 bis 300 000 g/mol und vorzugsweise 1 200 bis 60 000 g/mol aufweist.

14. Zusammensetzung nach einem der Ansprüche 1 bis 13, wobei der hydrophobe Block B eine Molmasse von 400 bis 200 000 g/mol und vorzugsweise 800 bis 40 000 g/mol besitzt.

15. Zusammensetzung nach einem der Ansprüche 1 bis 14, wobei der Masseanteil des ionischen hydrophilen Blocks A in dem Zweiblockpolymer A-B größer 60 % und insbesondere größer 70 % ist.

16. Zusammensetzung nach einem der Ansprüche 1 bis 14, wobei der Masseanteil des hydrophilen ionischen Blocks A in den Dreiblockpolymeren B-A-B der Erfindung größer 50 % und insbesondere größer 60 % ist.

17. Zusammensetzung nach einem der Ansprüche 1 bis 16, wobei die Zweiblock- oder Dreiblockpolymere einen ionischen wasserlöslichen Polymerblock A, der vollständig wasserlöslich ist, und hydrophobe Polymerblöcke B, die vollständig hydrophob sind, aufweisen.

18. Zusammensetzung nach einem der vorhergehenden Ansprüche, wobei die Zweiblockpolymere unter den Zweiblockpolymeren Polystyrol/Natriumpolyacrylat ausgewählt sind.

19. Zusammensetzung nach Anspruch 18, wobei das Zweiblockpolymer ausgewählt ist unter:
- dem Zweiblockpolymer Polystyrol (2 000 g/mol)-Natriumpolyacrylat (11 500 g/mol), wobei der wasserlösliche ionische Block (Natriumpolyacrylat) 85,2 % des Gesamtgewichts des Zweiblockpolymers ausmacht;
- dem Zweiblockpolymer Polystyrol (1 800 g/mol)-Natriumpolyacrylat (42 450 g/mol), wobei der wasserlösliche ionische Block (Natriumpolyacrylat) 95,9 % des Gesamtgewichts des Zweiblockpolymers ausmacht;
- dem Zweiblockpolymer Polystyrol (4 300 g/mol)-Natriumpolyacrylat (25 460 g/mol), wobei der wasserlösliche ionische Block 85,55 % des Gesamtgewichts des Zweiblock-Copolymers ausmacht.

20. Zusammensetzung nach einem der vorhergehenden Ansprüche, wobei die Dreiblockpolymere unter den Dreiblockpolymeren Polystyrol/Natriumpolyacrylat/Polystyrol ausgewählt sind.

21. Zusammensetzung nach Anspruch 20, wobei das Dreiblockpolymer das Polystyrol (2 500 g/mol)-Natriumpolyacrylat (29 800 g/mol)- Polystyrol (2 500 g/mol)-Dreiblockpolymer ist, wobei der Mengenanteil des wasserlöslichen Blocks 85,63 % des Gesamtgewichts des Dreiblock-Copolymers ausmacht.

22. Zusammensetzung nach einem der vorhergehenden Ansprüche,
wobei die Massekonzentration des Zweiblockpolymers oder Dreiblockpolymers in der Zusammensetzung vorzugsweise im Bereich von 0,01 bis 30 Gew.-% und bevorzugt 0,1 bis 20 Gew.-%, bezogen auf das Gesamtgewicht der Zusammensetzung, liegt.

23. Zusammensetzung nach einem der Ansprüche 1 bis 22, wobei das UV-Filter vom 4,4-Diarylbutadientyp der folgenden Formel (II) entspricht: 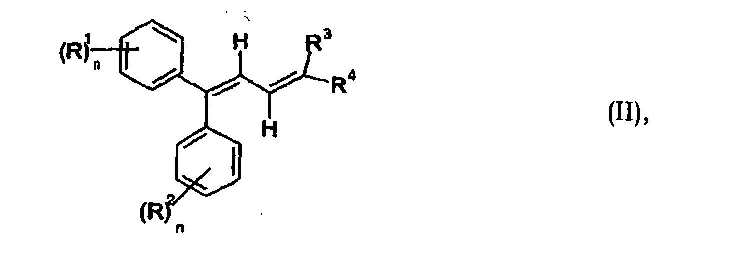 wobei das Diensystem die Konfiguration Z,Z; Z,E; E,Z oder E,E hat oder Gemische dieser Konfigurationen bedeutet und worin:
- die Gruppen R¹ und R², die gleich oder verschieden sind, Wasserstoff, C₁₋₂₀-Alkyl; C₂₋₁₀-Alkenyl; C₁₋₁₂-Alkoxy; C₃₋₁₀-Cycloalkyl; C₃₋₁₀-Cycloalkenyl; C₁₋₂₀-Alkoxycarbonyl; C₁₋₁₂-Monoalkylamino; C₁₋₁₂-Dialkylamino; Aryl; Heteroaryl oder einen wasserlöslichen Substituenten bedeutet, der unter Carboxylat, Sulfonat oder Ammonium ausgewählt ist;
- R³ eine Gruppe COOR⁵; COR⁵; CONR⁵R⁶; CN; O=S(-R⁵)=O; O=S(-OR⁵)=O; R⁷O-P-(-OR⁸)=O; C₁₋₂₀-Alkyl; C₂₋₁₀-Alkenyl; C₃₋₁₀-Cycloalkyl; C₇₋₁₀-Bicycloalkyl; C₃₋₁₀-Cycloalkenyl; C₇₋₁₀-Bicycloalkenyl; C₆₋₁₈-Aryl, wobei diese Gruppe gegebenenfalls substituiert ist; C₃₋₇-Heteroaryl bedeutet, wobei diese Gruppe gegebenenfalls substituiert ist;
- R⁴ eine Gruppe COOR⁶; COR⁶; CONR⁵R⁶; CN; O=S(-R⁶)=O; O=S(-OR⁶)=O; R⁷O-P-(-OR⁸)=O; C₁₋₂₀-Alkyl; C₂₋₁₀-Alkenyl; C₃₋₁₀-Cycloalkyl; C₇₋₁₀-Bicycloalkyl; C₃₋₁₀-Cycloalkenyl; C₇₋₁₀-Bicycloalkenyl; C₆₋₁₈-Aryl, wobei diese Gruppe gegebenenfalls substituiert ist; C₃₋₇-Heteroaryl bedeutet, wobei diese Gruppe gegebenenfalls substituiert ist;
- die Gruppen R⁵ bis R⁸, die gleich oder verschieden sind, Wasserstoff; C₁₋₂₀-Alkyl; C₂₋₁₀-Alkenyl; C₃₋₁₀-Cycloalkyl; C₇₋₁₀-Bicycloalkyl; C₃₋₁₀-Bicycloalkenyl; C₇₋₁₀-Cycloalkenyl; Aryl, wobei diese Gruppe gegebenenfalls substituiert ist; Heteroaryl bedeutet, wobei diese Gruppe gegebenenfalls substituiert ist;
- n im Bereich von 1 bis 3 liegt,
wobei die Gruppen R³ bis R⁸ mit den Kohlenstoffatomen, an die sie gebunden sind, einen C₅₋₆-Ring bilden können, der kondensiert sein kann.

24. Zusammensetzung nach Anspruch 23, wobei die Verbindung der Formel (II) unter den Verbindungen ausgewählt ist, worin bedeuten:
- n = 1 oder 2;
- R¹ und R², die gleich oder verschieden sind, Wasserstoff, C₁-₂₀-Alkyl; C₁₋₁₂-Alkoxy; C₁₋₁₂-Monoalkylamino; C₁₋₁₂-Dialkylamino; einen wasserlöslichen Substituenten, der unter Carboxylat, Sulfonat oder Ammonium ausgewählt ist;
- R³ COOR⁵; COR⁵; CONR⁵R⁶; C₁₋₂₀-Alkyl; C₃₋₁₀-Cycloalkyl; C₃₋₁₀-Cycloalkenyl, C₇₋₁₀-Bicycloalkyl; Phenyl, Naphthyl oder Thienyl, die gegebenenfalls substituiert sind;
- R⁴ eine Gruppe COOR⁶; COR⁶; CONR⁵R⁶; C₁₋₂₀-Alkyl; C₃₋₁₀-Cycloalkyl; C₃₋₁₀-Cycloalkenyl; C₇₋₁₀-Bicycloalkyl; Phenyl, Naphthyl oder Thienyl, die gegebenenfalls substituiert sind;
- die Gruppen R⁵ und R⁶, die gleich oder verschieden sind, Wasserstoff; C₁₋₁₂-Alkyl; C₃₋₁₀-Cycloalkyl; C₃₋₁₀-Cycloalkenyl ; C₇₋₁₀-Bicycloalkyl; C₃₋₁₀-Bicycloalkenyl; Phenyl oder Naphthyl, die gegebenenfalls substituiert sind.

25. Zusammensetzung nach Anspruch 24, wobei die Verbindung der Formel (II) unter den Verbindungen ausgewählt ist, worin bedeuten:
- R¹ und R², die gleich oder verschieden sind, Wasserstoff, C₁₋₂₀-Alkyl; C₁₋₂₀-Alkoxy; einen wasserlöslichen Substituenten, der unter Carboxylat, Sulfonat oder Ammonium ausgewählt ist;
- R³ COOR⁵; COR⁵; CONR⁵R⁶;
- R⁴ COOR⁶; COR⁶; CONR⁵R⁶;
- die Gruppen R⁵ und R⁶, die gleich oder verschieden sind, Wasserstoff; C₁₋₁₂-Alkyl, C₃₋₆-Cycloalkyl; C₃₋₁₀-Cycloalkenyl; C₇₋₁₀-Bicycloalkyl; C₃₋₁₀-Bicycloalkenyl; Phenyl oder Naphthyl, die gegebenenfalls substituiert sind.

26. Zusammensetzung nach Anspruch 25, wobei die Verbindung der Formel (I) unter den Verbindungen der folgenden Formel (II') ausgewählt ist: 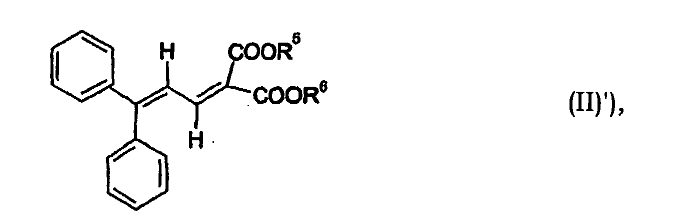 worin die Gruppen R⁵ und R⁶, die gleich oder verschieden sind, Wasserstoff; C₁₋₂₀-Alkyl; C₃₋₆-Cycloalkyl; C₃₋₁₀-Cycloalkenyl bedeuten.

27. Zusammensetzung nach Anspruch 26, wobei die Verbindung der Formel (II') das 1,1-(2',2'-Dimethylpropyl)dicarboxy-4,4-diphenyl-butadienderivat der folgenden Struktur ist:

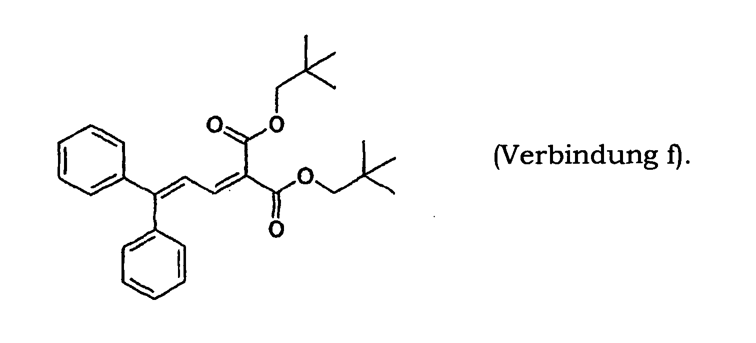

28. Zusammensetzung nach einem der Ansprüche 1 bis 22, wobei das UV-A-Filter vom 4,4-Diarylbutadientyp der folgenden Formel (III) entspricht: 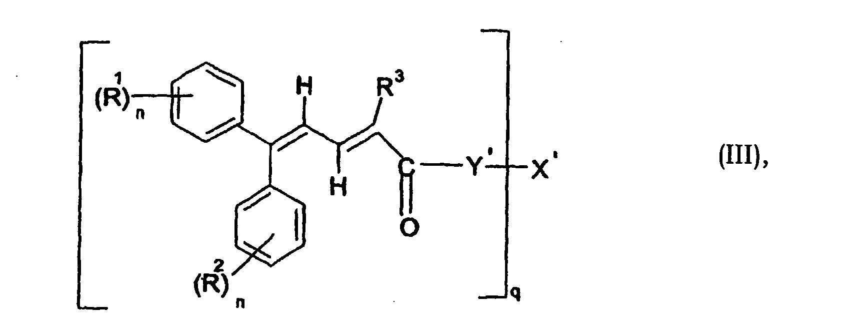 wobei das Diensystem die Konfiguration Z,Z; Z,E; E,Z oder E,E hat oder Gemische dieser Konfigurationen bedeutet und worin:
- R¹, R², R³ und n die in der Formel (II) angegebenen Bedeutungen aufweisen, wie sie in Anspruch 23 definiert sind;
- Y' eine Gruppe -O- oder -NR⁹- bedeutet;
- R⁹ Wasserstoff; eine geradkettige oder verzweigte C₁₋₂₀-Alkylgruppe; C₂₋₁₀-Alkenyl, C₃₋₁₀-Cycloalkyl; C₇₋₁₀-Bicycloalkyl; C₃₋₁₀-Cycloalkenyl; C₇₋₁₀-Bicycloalkenyl; Aryl; Heteroaryl bedeutet;
- X' einen geradkettigen oder verzweigten, aliphatischen oder cycloaliphatischen C₂₋₂₀-Polyolrest bedeutet, der 2 bis 10 Hydroxygruppen enthält und die Valenz q hat; wobei die Kohlenstoffkette dieses Rests durch ein oder mehrere Schwefelatome oder Sauerstoffatome unterbrochen sein kann; eine oder mehrere Imingruppen; eine oder mehrere Alkyliminogruppen mit 1 bis 4 Kohlenstoffatomen bedeutet;
- q im Bereich von 2 bis 10 liegt.

29. Zusammensetzung nach Anspruch 28, wobei die Verbindung der Formel (II) unter den Verbindungen ausgewählt ist, worin bedeuten:
- R¹ und R², die gleich oder verschieden sind, Wasserstoff, C₁₋₁₂-Alkyl; C₁₋₈-Alkoxy; einen wasserlöslichen Substituenten, der unter Carboxylat, Sulfat oder Ammonium ausgewählt ist;
- R³ eine Gruppe COOR⁵; CONR⁵R⁶; CN; C₃₋₁₀-Cycloalkyl; C₇₋₁₀-Bicycloalkyl;
- R⁵ und R⁶, die gleich oder verschieden sind, eine geradkettige oder verzweigte C₁₋₂₀-Alkylgruppe; C₃₋₁₀-Cycloalkyl; C₇₋₁₀-Bicycloalkyl; Naphthyl oder Phenyl, die gegebenenfalls substituiert sind;
- X' einen C₂₋₂₀-Polyolrest, der 2 bis 6 Hydroxygruppen und insbesondere 2 bis 4 Hydroxygruppen enthält.

30. Zusammensetzung nach Anspruch 29, wobei die Verbindung der Formel (III) unter den Verbindungen ausgewählt ist, worin X' einen Ethanolrest oder Pentaerythritrest bedeutet.

31. Zusammensetzung nach Anspruch 30, wobei die Verbindung der Formel (III) unter den folgenden Verbindungen ausgewählt ist:

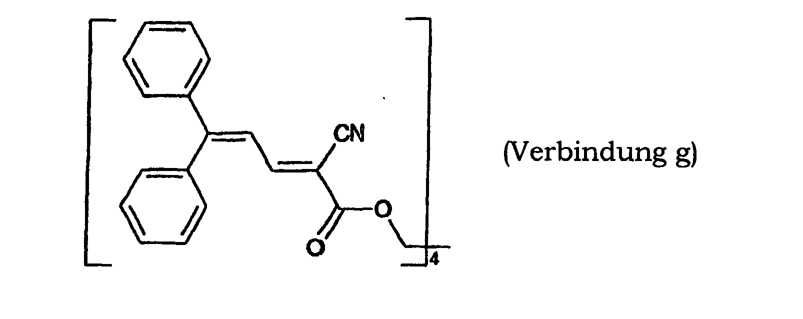

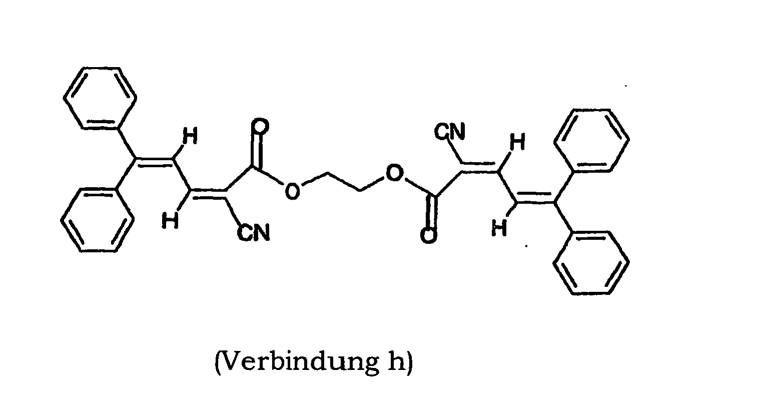

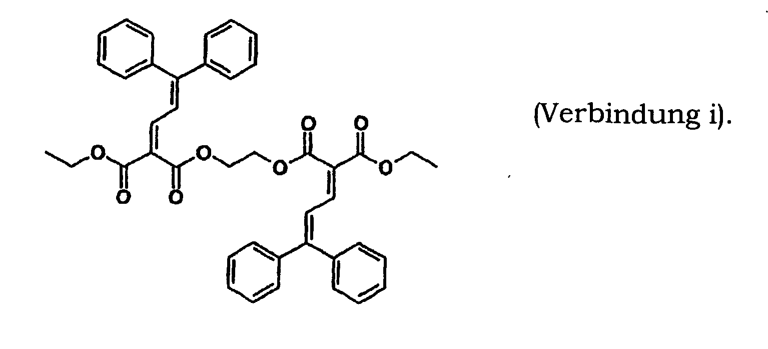

32. Zusammensetzung nach einem der Ansprüche 1 bis 31, **dadurch gekennzeichnet, dass** das oder die 4,4-Diarylbutadien(e) in Mengenanteilen von 0,1 bis 20 Gew.-% und vorzugsweise 1 bis 10 Gew.-%, bezogen auf das Gesamtgewicht der Zusammensetzung, enthalten sind.

33. Zusammensetzung nach einem der Ansprüche 1 bis 32, **dadurch gekennzeichnet, dass** sie ferner mindestens ein ergänzendes, organisches oder anorganisches Sonnenschutzfilter enthält, das im UV-A- und/oder UV-B-Bereich wirksam und wasserlöslich, fettlöslich oder in gewöhnlich verwendeten kosmetischen Lösungsmitteln unlöslich ist.

34. Zusammensetzung nach Anspruch 33, wobei die zusätzlichen organischen Filter unter den Anthranilaten; Zimtsäurederivaten; Dibenzoylmethanderivaten; Salicylsäurederivaten; Campherderivaten; Triazinderivaten; Benzophenonderivaten; β-β-Diphenylacrylatderivaten; Benzotriazolderivaten; Benzalmalonatderivaten; Benzimidazolderivaten; Imidazolinen; Bisbenzoazolylderivaten; p-Aminobenzoesäurederivaten (PABA); Benzoxazolderivaten; Methylen-bis(hydroxyphenylbenzotriazol)derivaten; polymeren Filtern und Siliconfiltern; von α-Alkylstyrol abgeleiteten Dimeren und deren Gemischen ausgewählt sind.

35. Zusammensetzung nach Anspruch 34, wobei die zusätzlichen organischen Filter ausgewählt sind unter:
Ethylhexyl Salicylate,
Ethylhexyl Methoxycinnamate,
Octocrylene,
Butyl Methoxydibenzoylmethane,
Phenylbenzimidazole Sulfonic Acid,
Benzophenone-3,
Benzophenone-4,
Benzophenone-5,
n-Hexyl-2-(4-diethylamino-2-hydroxybenzoyl)-benzoat,
4-Methylbenzylidene camphor,
Terephthalylidene Dicamphor Sulfonic Acid,
Disodium Phenyl Dibenzimidazole Tetra-sulfonate,
2,4,6-Tris-(diisobutyl-4'-aminobenzalmalonat)-s-triazin,
Anisotriazine,
Ethylhexyl Triazone,
Diethylhexyl Butamino Triazone,
Methylene bis-Benzotriazolyl Tetramethylbutylphenol,
Drometrizole Trisiloxane,
Polysilicone-15,
2,4-Bis[5-1-(dimethylpropyl)benzoxazol-2-yl-(4-phenyl)-imino]-6-(2-ethylhexyl)-imino-1,3,5-triazin und deren Gemischen.

36. Zusammensetzung nach Anspruch 33, wobei die zusätzlichen anorganischen Filter unter den Pigmenten oder Nanopigmenten von Metalloxiden, die gegebenenfalls umhüllt sind, ausgewählt sind.

37. Zusammensetzung nach Anspruch 36, wobei die zusätzlichen anorganischen Filter unter den Nanopigmenten von amorphem oder kristallisiertem Titanoxid in Form von Rutil und/oder Anatas, von Eisenoxid, Zinkoxid, Zirconiumoxid oder Ceroxid ausgewählt sind.

38. Zusammensetzung nach einem der Ansprüche 1 bis 37, **dadurch gekennzeichnet, dass** sie ferner mindestens ein Bräunungsmittel und/oder Mittel zur künstlichen Braunfärbung der Haut enthält.

39. Zusammensetzung nach einem der Ansprüche 1 bis 38, **dadurch gekennzeichnet, dass** sie ferner mindestens einen kosmetischen Zusatzstoff enthält, der unter den organischen Lösungsmitteln, ionischen oder nichtionischen Verdickungsmitteln, beruhigenden Stoffen, Feuchthaltemitteln, Wirkstoffen, Trübungsmitteln, Stabilisatoren, Emollientien, Siliconen, insektenabwehrenden Stoffen, Parfums, Konservierungsmitteln, grenzflächenaktiven Stoffen, Füllstoffen, Pigmenten, Polymeren, Treibmitteln, Alkalisierungsmitteln oder Ansäuerungsmitteln oder beliebigen anderen, gewöhnlich in der Kosmetik und/oder Dermatologie verwendeten Bestandteilen ausgewählt ist.

40. Zusammensetzung nach einem der Ansprüche 1 bis 39, **dadurch gekennzeichnet, dass** sie als Lotion oder Serum ohne Fettphase, Öl-in-Wasser-Emulsion oder Wasser-in-Öl-Emulsion; multiple Emulsion, Mikroemulsion, Vesikeldispersion vom ionischen und/oder nichtionischen Typ oder Wachs/wässrige Phase-Dispersion vorliegt.

41. Zusammensetzung nach einem der Ansprüche 1 bis 40, **dadurch gekennzeichnet, dass** sie als Öl-in-Wasser-Emulsion oder Wasser-in-Öl-Emulsion vorliegt, die höchstens 1 Gew.-% eines emulgierenden grenzflächenaktiven Stoffes, bezogen auf das Gesamtgewicht der Zusammensetzung, enthält.

42. Verwendung einer Zusammensetzung nach einem der Ansprüche 1 bis 41 für die Herstellung von Produkten für die kosmetische Behandlung der Haut, der Lippen und der Haare einschließlich der Kopfhaut, insbesondere zum Schutz und/oder zur Pflege der Haut, der Lippen und/oder der Haare und/oder zum Schminken der Haut und/ oder der Lippen.
